# EUROPEAN PATENT SPECIFICATION

(11) **EP 4 314 318 B1**
(45) Date of publication and mention of the grant of the patent: **28.08.2024**
(21) Application number: 22720575.4
(22) Date of filing: 31.03.2022
(51) Int. Cl.: C12Q 1/18

(54) **A METHOD FOR IDENTIFYING A COMPOSITION OR COMPOUND HAVING ANTIMICROBIAL AND/OR ANTI-BIOFILM ACTIVITIES AGAINST A MICROORGANISM OF INTEREST**
VERFAHREN ZUR IDENTIFIZIERUNG EINER ZUSAMMENSETZUNG ODER VERBINDUNG MIT ANTIMIKROBIELLER UND/ODER ANTI-BIOFILM-AKTIVITÄT GEGEN EINEN BESTIMMTEN MIKROORGANISMUS
PROCÉDÉ D'IDENTIFICATION D'UNE COMPOSITION OU D'UN COMPOSÉ AYANT DES ACTIVITÉS ANTIMICROBIENNES ET/OU ANTI-BIOFILM CONTRE UN MICRO-ORGANISME D'INTÉRÊT

(30) Priority: 01.04.2021 LU 102767
(43) Date of publication of application: 07.02.2024
(73) Proprietor: Univerza V Ljubljani, 1000 Ljubljana (SI); Jozef Stefan Institute, 1000 Ljubljana (SI)
(72) Inventor: STERNISA, Meta, 1000 Ljubljana (SI); SABOTIC, Jerica, 1000 Ljubljana (SI); KLANCNIK, Anja, 1000 Ljubljana (SI)
(74) Representative: Zacco GmbH
(86) International application number: PCT/EP2022/058548
(87) International publication number: WO 2022/207781

(56) References cited:
- WO-A1-2014/145899
- WO-A2-2007/050513
- SCHUMACHER A ET AL: "In vitro antimicrobial susceptibility testing methods: agar dilution to 3D tissue-engineered models", EUROPEAN JOURNAL OF CLINICAL MICROBIOLOGY & INFECTIOUS DISEASES, vol. 37, no. 2, 2017, pages 187 - 208, XP036400935

## Description

### Technical field of the invention

The present invention generally relates to the field of microbiology, and more specifically to the field of antimicrobials. The present invention is based on a novel approach for searching active compositions or compounds having antimicrobial and/or anti-biofilm activities against a microorganism of interest based on growth curve analysis. The present invention provides a method for identifying a composition or compound having antimicrobial and/or anti-biofilm activities against a microorganism of interest, comprising the steps of a) providing a composition or compound, b) bringing the composition or compound in contact with a microorganism of interest in a growth medium; c) incubating the microorganism in the presence of the composition or compound; d) determining the growth of the microorganism by measuring the optical density in intervals during the incubation under c); e) preparing a growth curve from the measurement obtained under d); and f) analyzing the growth curve to determine if the composition or compound has antimicrobial and/or anti-biofilm activities against the microorganism;
wherein step f) comprises comparing the growth curve prepared in step e) with a control growth curve which is obtained in accordance with steps c) to e) with all conditions being identical except that the microorganism is incubated in the absence of the composition or compound;
wherein the control growth curve exhibits a first phase including an increase in optical density within a first period of time, a second phase including an increase in optical density within a second period of time, and a stationary phase between the first phase and the second phase.

### Background of the invention

Biofilms represent a typical lifestyle of microorganisms in the environment. Biofilms are microbial communities encased within a self-produced matrix that provides numerous advantages, such as physical protection from the host immune system and environmental factors (e.g. UV light, acids, salinity, antibiotics, sanitizers), as well as retention of water, storage of nutrients and exchange of genetic information enabling spread of antimicrobial resistance. Therefore, biofilms aggravate the occurrence of antimicrobial resistance and provide a continuous source of contamination, which also applies to *Salmonella* [1,2]. Biofilm formation is a persistence strategy for microorganisms, and has a role for both biotic surfaces, to cause chronic infections, and for colonisation of abiotic surfaces. After adhesion to different surfaces, *Salmonella* can form a protective matrix of extracellular polymeric substances (EPS) that is composed of polysaccharides, proteins, nucleic acids and lipids [2]. These protect the microorganisms from environmental factors, facilitate gene transfer, and increase availability of nutrients and microbial resistance to antimicrobial agents. The better adaptability of *Salmonella* biofilm cells compared to free-floating planktonic cells additionally contributes to environmental survival and increased antimicrobial resistance, both of which are related to infections in humans [1,3,4]. *Salmonella* spp. can adhere to abiotic surfaces like plastic, glass and stainless steel, and to plant surfaces and other biotic surfaces, such as epithelial cells and gallstones, where they can then form biofilms [5].

According to the WHO, food-borne illnesses represent a global public health problem that affects an estimated 600 million people annually (WHO, 2020). In the European Union, salmonellosis is the second most common food-borne zoonosis and the most commonly identified cause of food-borne outbreaks, where transmission through various food sources can result in human contamination (mainly from pork, poultry, beef, fresh produce). The most commonly reported isolates are S. Enteritidis, S. Typhimurium, S. Infantis and S. Newport [6]. In addition, *Salmonella* isolated from different food sources show multidrug resistance [7], which poses a high risk to public health. These resistant *Salmonella* are also widespread in animals and on various surfaces related to animal production and food processing [4,8,9]. Due to resistance to environmental stress and high resistance to commercially available antimicrobial agents, alternative control strategies for these *Salmonella* are being sought.

Much attention is being given to natural sources of active compounds that might have anti-*Salmonella* activities - for both antimicrobial and anti-biofilm agents. Investigations have included various extracts from plants, fungi or processing wastes, interactions with other microorganisms or their products, and use of bacteriophages [10-14]. Despite extensive research, however, *Salmonella* resistance and biofilm formation remain a problem. As antimicrobial agents, these compounds inhibit bacterial growth or kill the bacteria; as specific anti-biofilm agents, they can interact with biofilm-related metabolic processes or quorum sensing without affecting bacterial growth [5]. These two concepts need to be treated separately, as compounds that affect growth and biofilms at the same time might also contribute to further development of bacterial resistance; therefore, there is the need to target biofilms and their formation without affecting bacterial growth [15]. Indeed, control of biofilm formation might represent a better option in the fight against bacteria, especially due to increasing occurrence of antimicrobial resistance. A Schumacher et al: European Journal of Clinical Microbiology & Infectious Diseases, vol. 37, no. 2, (2017), pages 187-208 discloses in vitro antimicrobial susceptibility testing methods ranging from agar dilution to 3D tissue-engineered models.

The search for new active compounds often requires extensive screening studies. For antimicrobial activities, considering the large number of tests available, fast screening microdilution methods [16] are the most accessible and most commonly used [17]. For anti-biofilm tests, classical microbiological methods (e.g., colony forming units, CFU) are used to determine cultivable biofilm cells, dye staining assays to detect biofilm biomass (e.g., crystal violet) and metabolic activity assays to evaluate viability of cells (e.g., resazurin, tetrazolium salts) [18]. The viability of biofilm cells can also be determined by molecular techniques (e.g., qPCR) [19,20] or by flow cytometry with different fluorophores [21,22]. Flow cytometry also allows assessment of cells in different physiological states. Various microscopy techniques can also be used to assess antimicrobial or anti-biofilm activities - from simple light microscopy to fluorescence and scanning electron microscopy. Despite these possibilities, none of these methods can simultaneously determine antimicrobial and anti-biofilm activities in a relatively short time.

Accordingly, there remains a need for improved methods for searching active compositions and compounds that have either antimicrobial or anti-biofilm properties or both, making them particularly useful as agents against *Salmonella* spp. or other microbial species.

### Summary of the invention

The present invention addresses this need by providing a novel approach for searching active compositions and compounds having antimicrobial and/or anti-biofilm activities against a microorgansim of interest based on growth curve analysis. The present invention allows an easy and rapid high-throughput analysis with simultaneous and distinguishable detection of antimicrobial and anti-biofilm activities.

The present invention thus provides a method for identifying a composition or compound having antimicrobial and/or anti-biofilm activities against a microorganism of interest, comprising the steps of a) providing a composition or compound, b) bringing the composition or compound in contact with a microorganism of interest in a growth medium; c) incubating the microorganism in the presence of the composition or compound; d) determining the growth of the microorganism by measuring the optical density in intervals during the incubation under c); e) preparing a growth curve from the measurement obtained under d); and f) analyzing the growth curve to determine if the composition or compound has antimicrobial and/or anti-biofilm activities against the microorgnism;
wherein step f) comprises comparing the growth curve prepared in step e) with a control growth curve which is obtained in accordance with steps c) to e) with all conditions being identical except that the microorganism is incubated in the absence of the composition or compound;
wherein the control growth curve exhibits a first phase including an increase in optical density within a first period of time, a second phase including an increase in optical density within a second period of time, and a stationary phase between the first phase and the second phase.

Particularly, the present invention provides a method for identifying a composition or compound having anti-bacterial and/or anti-biofilm activities against a bacterium of interest, comprising the steps of a) providing a compound, b) bringing the composition or compound in contact with a bacterium of interest in a growth medium; c) incubating the bacterium in the presence of the composition or compound; d) determining the bacterial growth by measuring the optical density in intervals during the incubation under c); e) preparing a growth curve from the measurement obtained under d); and f) analyzing the growth curve to determine if the composition or compound has antibacterial and/or anti-biofilm activities against the bacterium;
wherein step f) comprises comparing the growth curve prepared in step e) with a control growth curve which is obtained in accordance with steps c) to e) with all conditions being identical except that the bacterium is incubated in the absence of the composition or compound;
wherein the control growth curve exhibits a first phase including an increase in optical density within a first period of time, a second phase including an increase in optical density within a second period of time, and a stationary phase between the first phase and the second phase.

The present invention may be further summarized by the following items:
1. A method for identifying a composition or compound having antimicrobial and/or anti-biofilm activities against a microorganism of interest, comprising the steps of a) providing a composition or compound, b) bringing the composition or compound in contact with a microorganism of interest in a growth medium; c) incubating said microorganism in the presence of said composition or compound; d) determining the growth of the microorganism by measuring the optical density in intervals during the incubation under c); e) preparing a growth curve from the measurement obtained under d); and f) analyzing the growth curve to determine if the composition or compound has antimicrobial and/or anti-biofilm activities against the microorganism;
   wherein step f) comprises comparing the growth curve prepared in step e) with a control growth curve which is obtained in accordance with steps c) to e) with all conditions being identical except that the microorganism is incubated in the absence of the composition or compound;
   wherein the control growth curve exhibits a first phase including an increase in optical density within a first period of time, a second phase including an increase in optical density within a second period of time, and a stationary phase between the first phase and the second phase.
2. The method according to item 1, wherein the composition or compound is brought in contact with the microorganism in step b) by mixing an inoculum of the microorganism in growth medium with the composition or compound.
3. The method according to item 2, wherein the inoculum of the microorganism in growth medium is mixed with the composition or compound with a final volume of up to 10 ml.
4. The method according to item 2, wherein the inoculum of the microorganism in growth medium is mixed with the composition or compound with a final volume of up to 1 ml.
5. The method according to item 2, wherein the inoculum of the microorganism in growth medium is mixed with the composition or compound with a final volume of 1 µ! to 10 ml.
6. The method according to item 2, wherein the inoculum of the microorganism in growth medium is mixed with the composition or compound with a final volume of 1 µ! to 1 ml.
7. The method according to item 2, wherein the inoculum of the microorganism in growth medium is mixed with the composition or compound with a final volume of 10 µl to 1 ml.
8. The method according to item 2, wherein the inoculum of the microorganism in growth medium is mixed with the composition or compound with a final volume of 50 to 500 µL.
9. The method according to item 2, wherein the inoculum of the microorganism in growth medium is mixed with the composition or compound with a final volume of 100 to 300 µl.
10. The method according to any one of items 2 to 9, wherein the final volume is 200 µL.
11. The method according to any one of items 2 to 10, wherein the inoculum of the microorganism has a concentration in a range from 10¹ to 10⁹ CFU/mL.
12. The method according to any one of items 2 to 11, wherein the inoculum of the microorganism has a concentration in a range from 10² to 10⁸ CFU/mL.
13. The method according to any one of items 2 to 12, wherein the inoculum of the microorganism has a concentration in a range from 10³ to 10⁷ CFU/mL.
14. The method according to any one of items 2 to 13, wherein the inoculum of the microorganism has a concentration in a range from 10⁴ to 10⁶ CFU/mL.
15. The method according to any one of items 2 to 14, wherein the inoculum of the microorganism has a concentration of 10⁵ CFU/mL.
16. The method according to any one of items 1 to 15, wherein the incubation in step c) is carried out for a suitable period of time.
17. The method according to any one of items 1 to 16, wherein the incubation in step c) is carried out for a period from 5 to 60 hours.
18. The method according to any one of items 1 to 17, wherein the incubation in step c) is carried out for a period from 10 to 50 hours.
19. The method according to any one of items 1 to 18, wherein the incubation in step c) is carried out for a period from 15 to 40 hours.
20. The method according to any one of items 1 to 19, wherein the incubation in step c) is carried out for a period from 15 to 25 hours.
21. The method according to any one of items 1 to 20, wherein the incubation in step c) is carried out for 20 hours.
22. The method according to any one of items 1 to 21, wherein the incubation in step c) is carried out at a suitable temperature.
23. The method according to any one of items 1 to 22, wherein the incubation in step c) is carried out at a temperature from 4°C to 70 °C.
24. The method according to any one of items 1 to 23, wherein the incubation in step c) is carried out at a temperature from 4°C to 60 °C.
25. The method according to any one of items 1 to 24, wherein the incubation in step c) is carried out at a temperature from 4° to 50°C.
26. The method according to any one of items 1 to 25, wherein the incubation in step c) is carried out at a temperature from 4° to 47 °C.
27. The method according to any one of items 1 to 26, wherein the incubation in step c) is carried out at a temperature from 20 to 47 °C.
28. The method according to any one of items 1 to 27, wherein the incubation in step c) is carried out at a temperature from room temperature to 40 °C.
29. The method according to any one of items 1 to 28, wherein the incubation in step c) is carried out at a temperature from 30 to 40°C.
30. The method according to any one of items 1 to 29, wherein the incubation in step c) is carried out at a temperature from 35 to 38 °C.
31. The method according to any one of items 1 to 30, wherein the incubation in step c) is carried out at 37 °C.
32. The method according to any one of items 1 to 31, wherein the incubation in step c) is carried out for 20 hours at 37 °C.
33. The method according to any one of items 1 to 32, wherein the optical density in step d) is measured at a suitable interval.
34. The method according to any one of items 1 to 33, wherein the optical density in step d) is measured in intervals of 10 seconds to 120 minutes.
35. The method according to any one of items 1 to 34, wherein the optical density in step d) is measured in intervals of 10 seconds to 60 minutes.
36. The method according to any one of items 1 to 35, wherein the optical density in step d) is measured in intervals of 30 seconds to 60 minutes.
37. The method according to any one of items 1 to 36, wherein the optical density in step d) is measured in intervals of 1 to 60 minutes.
38. The method according to any one of items 1 to 37, wherein the optical density in step d) is measured in intervals of 1 to 45 minutes.
39. The method according to any one of items 1 to 38, wherein the optical density in step d) is measured in intervals of 10 to 30 minutes.
40. The method according to any one of items 1 to 39, wherein the optical density in step d) is measured in intervals every 1, 2, 5, 10, 15, 20, 25, 30, 35, 40, 45, 50, 55 or 60 minutes.
41. The method according to any one of items 1 to 40, wherein the optical density in step d) is measured in intervals every 10, 15, 20 or 30 minutes.
42. The method according to any one of items 1 to 41, wherein the optical density in step d) is measured in intervals every 30 minutes.
43. The method according to any one of items 1 to 42, wherein steps b) and c) are carried out in a suitable reaction vessel.
44. The method according to any one of items 1 to 43, wherein steps b) and c) are carried out in a test tube or flask.
45. The method according to any one of items 1 to 44, wherein steps b) and c) are carried out in a test tube.
46. The method according to item 44 or 45, wherein the test tube is a well of a microtiter plate.
47. The method according to any one of items 1 to 46, wherein the optical density is measured using a spectrometer or densitometer.
48. The method according to any one of items 1 to 47, wherein the optical density is measured using a spectrometer.
49. The method according to item 47 or 48, wherein the spectrometer is a UV spectrometer or a UV-VIS spectrometer.
50. The method according to any one of items 1 to 49, wherein the optical density is measured using a UV spectrometer.
51. The method according to any one of items 1 to 50, wherein the optical density in step d) is measured at wavelength from 500 to 700 nm.
52. The method according to any one of items 1 to 51, wherein the optical density in step d) is measured at wavelength from 550 to 650 nm.
53. The method according to any one of items 1 to 52, wherein the optical density in step d) is measured at wavelength of 600 nm.
54. The method according to any one of items 1 to 53, wherein the growth curve is prepared with time on the abscissa axis and the measurement obtained under d) on the ordinate axis.
55. The method according to any one of items 1 to 54, wherein step f) comprises comparing the growth curve prepared in step e) with a control growth curve.
56. The method according to item 55, wherein the control growth curve is obtained in accordance with steps c) to e) with all conditions being identical except that the microorganism is incubated in the absence of the composition or compound.
57. The method according to item 55 or 56, wherein the control growth curve exhibits a first phase including an increase in optical density within a first period of time and a second phase including an increase in optical density within a second period of time.
58. The method according to item 57, wherein the first period of time corresponds to the first half of the incubation time under c) and the second period corresponds to the second half of the incubation time under c).
59. The method according to item 57, wherein the first period of time corresponds to the first 10 hours of the incubation time under c) and the second period corresponds to the remaining incubation time.
60. The method according to any one of items 57 to 59, wherein the control growth curve further exhibits a stationary phase between the first phase and the second phase.
61. The method according to any one of items 57 to 60, wherein the composition or compound is considered having antimicrobial activity against the microorganism if the growth curve, when compared to the control growth curve, shows a decrease in the optical density in the first phase within the first period of time.
62. The method according to any one of items 57 to 61, wherein the composition or compound is considered having antimicrobial activity against the microorganism if the growth curve, when compared to the control growth curve, shows a decrease of at least 10% in the optical density in the first phase within the first period of time.
63. The method according to any one of items 57 to 62, wherein step f) comprises evaluating at least one growth parameter for the first phase.
64. The method according to any one of items 57 to 63, wherein step f) comprises evaluating at least two growth parameters for the first phase.
65. The method according to any one of items 57 to 64, wherein step f) comprises evaluating at least three growth parameters for the first phase.
66. The method according to any one of items 63 to 65, wherein the at least one growth parameter is selected from the group consisting of lag phase length, carrying capacity, growth rate, doubling time, time in which the population density reaches half of the carrying capacity, and end-point optical density (OD).
67. The method according to any one of items 63 to 66, wherein the composition or compound is considered having antimicrobial activity against the microorganism if at least one parameter in the growth curve selected from the group consisting of carrying capacity, growth rate, and end-point optical density (OD) is lower than the corresponding parameter in the control growth curve.
68. The method according to any one of items 63 to 67, wherein the composition or compound is considered having antimicrobial activity against the microorganism if at least one parameter in the growth curve selected from the group consisting of carrying capacity, growth rate, and end-point optical density (OD) is decreased by at least 10% compared to the corresponding parameter in the control growth curve.
69. The method according to any one of items 63 to 68, wherein the composition or compound is considered having antimicrobial activity against the microorganism if at least one parameter in the growth curve selected from the group consisting of the lag phase length, doubling time, and time in which the population density reaches half of the carrying capacity is longer than the corresponding parameter in the control growth curve.
70. The method according to any one of items 63 to 69, wherein the composition or compound is considered having antimicrobial activity against the microorganism if at least one parameter in the growth curve selected from the group consisting of the lag phase length, doubling time, and time in which the population density reaches half of the carrying capacity is increased by at least 10% compared to the corresponding parameter in the control growth curve.
71. The method according to any one of items 57 to 70, wherein the composition or compound is considered having anti-biofilm activity against the microorganism if the growth curve, when compared to the control growth curve, shows a decrease in the optical density in the second phase within the second period of time.
72. The method according to any one of items 57 to 71, wherein the composition or compound is considered having anti-biofilm activity against the microorganism if the growth curve, when compared to the control growth curve, shows a decrease of at least 10% in the optical density in the second phase within the second period of time.
73. The method according to any one of items 57 to 72, wherein step f) further comprises evaluating the end-point optical density (OD) for the second phase.
74. The method according to item 73, wherein the composition or compound is considered having anti-biofilm activity against the microorganism if the end-point OD in the growth curve is lower than the end-point OD in the control growth curve.
75. The method according to any one of items 1 to 74, wherein step f) comprises a statistical analysis of the growth curve.
76. The method according to any one of items 1 to 75, wherein the microorganism of interest is a bacterium or a yeast.
77. The method according to any one of items 1 to 76, wherein the microorganism of interest is a bacterium.
78. The method according to item 76 or 77, wherein the bacterium is selected from the group consisting of food-borne pathogenic bacteria, medically important bacteria including clinically important bacteria, bacteria important to dental health, bacteria important to veterinary medicine and environmentally important bacteria.
79. The method according to any one of items 76 to 78, wherein the bacterium is a gram-negative bacterium.
80. The method according to any one of items 76 to 79, wherein the bacterium is a bacterium of the family Enterobacteriaceae.
81. The method according to item 76 or 77, wherein the bacterium is a bacterium of the genus *Listeria, Escherichia, Yersinia, Vibrio, Salmonella, Pseudomonas, Staphylococcus, Streptococcus, Bacillus, Aeromonas, Campylobacter* or *Shigella.*
82. The method according to item 76 or 77, wherein the bacterium is a bacterium of the genus *Salmonella.*
83. The method according to any one of items 76 or 77, wherein the bacterium is selected from the group consisting of: *Listeria monocytogenes, Listeria innocua, Yersinia enterocolitica, Vibrio parachaemoliticus, Escherichia coli, Salmonella enterica,* including *Salmonella* Enteritidis and *Salmonella enterica* subps. *enterica* serovar Infantis *(Salmonella* Infantis), *Staphylococcus cures, Staphylococcus epidermidis, Streptococcus pyogenes, Pseudomonas aeruginosa, Pseudomonas fluorescens, Bacillus subtilis, Bacillus cereus, Aeromonas hydrophila, Aeromonas salmonicida, Campylobacter jejuni, Campylobacter coli* and *Shigella dysenteriae.*
84. The method according to any one of items 76 or 77, wherein the bacterium is *Salmonella* Infantis.
85. The method according to any one of items 1 to 76, wherein the microorganism of interest is a yeast.
86. The method according to item 76 or 85, wherein the yeast is a yeast of the genus *Candida, Cryptococcus, Pichia* or *Saccharomyces.*
87. The method according to item 76 or 85, wherein the yeast is selected from the group consisting of: *Candida albicans, Candida auris, Candida glabrata, Candida parapsilosis, Candida tropicalis, Candida krusei,* and *Cryptococcus neoformans.*
88. The method according to any one of items 1 to 87, wherein the composition is derived from a natural source.
89. The method according to any one of items 1 to 87, wherein the composition is an extract or a fraction thereof derived from a fungus, plant, algae, archaea, bacterium or animal cell or tissue.
90. The method according to any one of items 1 to 87, wherein the composition is an extract or a fraction thereof derived from a fungus.
91. The method according to any one of items 1 to 87, wherein the composition is an extract or a fraction thereof derived from a bacterium.
92. The method according to any one of items 1 to 87, wherein the composition comprises one or more chemically synthesized compounds.
93. The method according to any one of items 1 to 87, wherein the compound is a chemically synthesized compound.
94. The method according to any one of items 1 to 87, wherein the compound has been isolated from a natural source.
95. The method according to any one of items 1 to 87, wherein the compound has been isolated from an extract or a fraction thereof derived from a fungus, plant, algae, archaea, bacterium or animal cell or tissue.
96. The method according to any one of items 1 to 87, wherein the compound has been isolated from an extract or a fraction thereof derived from a fungus.
97. The method according to any one of items 1 to 87, wherein the compound has been isolated from an extract or a fraction thereof derived from a bacterium.

### Brief description of the figures

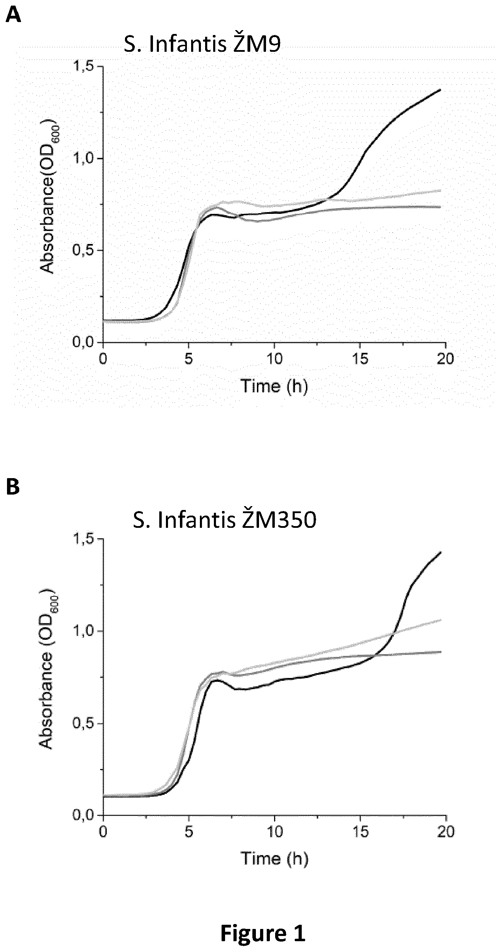

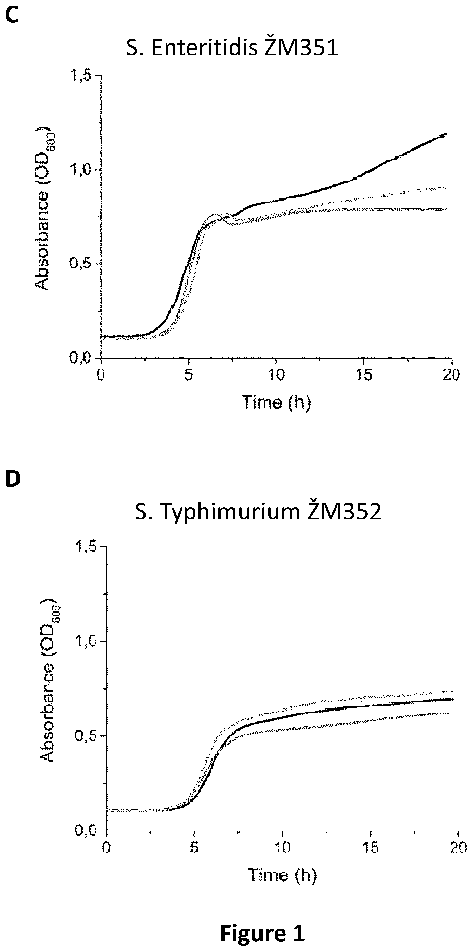

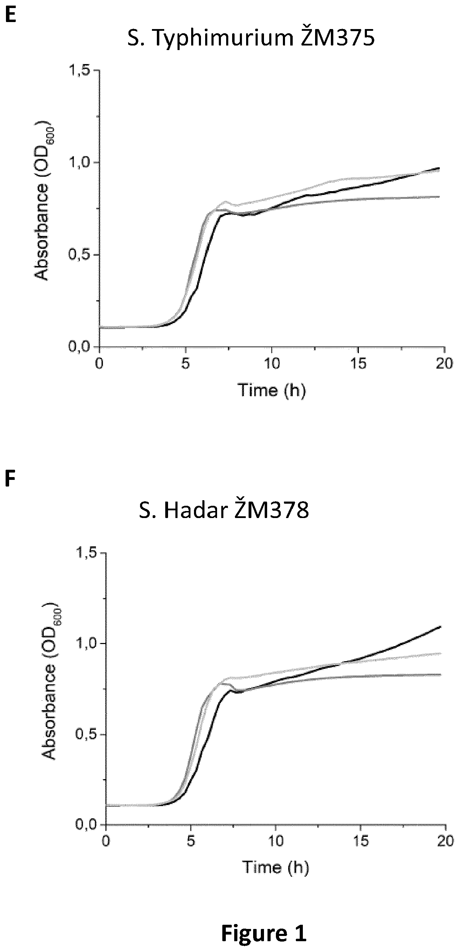

**Figure 1****.** Growth curves for the *Salmonella enterica* subps. *enterica* serovars (as indicated; black), and the influence of the *Pleurotus ostreatus* extract at relative concentration 50% (dilution of 1:1; dark grey) and 6.25% (dilution of 1:16; light grey).

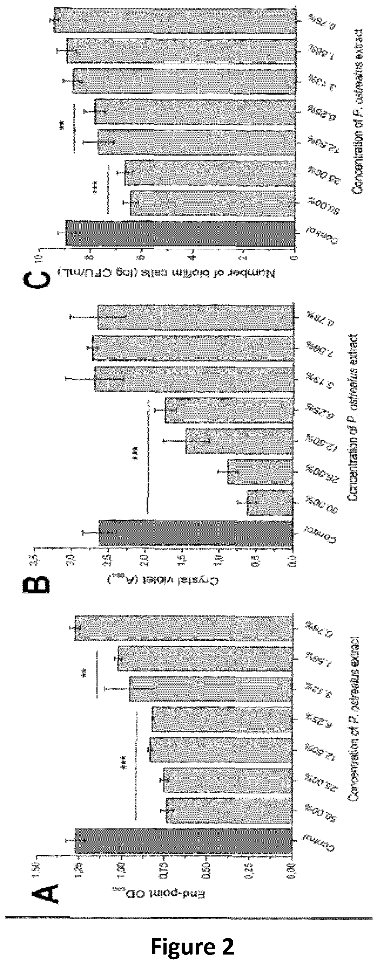

**Figure 2****.** Influence of the relative concentrations (serial dilutions from 1:1 to 1:128) of the *Pleurotus ostreatus* extract on the *Salmonella* Infantis ŽM9 end-point optical density (A), biofilm biomass (crystal violet assay) (B) and number of biofilm cells (C). Data are means ± standard deviation. *, *p* <0.05; **, *p* <0.01; *** *p* <0.001; <control (Dunnett's t-tests). Horizontal lines: significance applies to all columns covered.

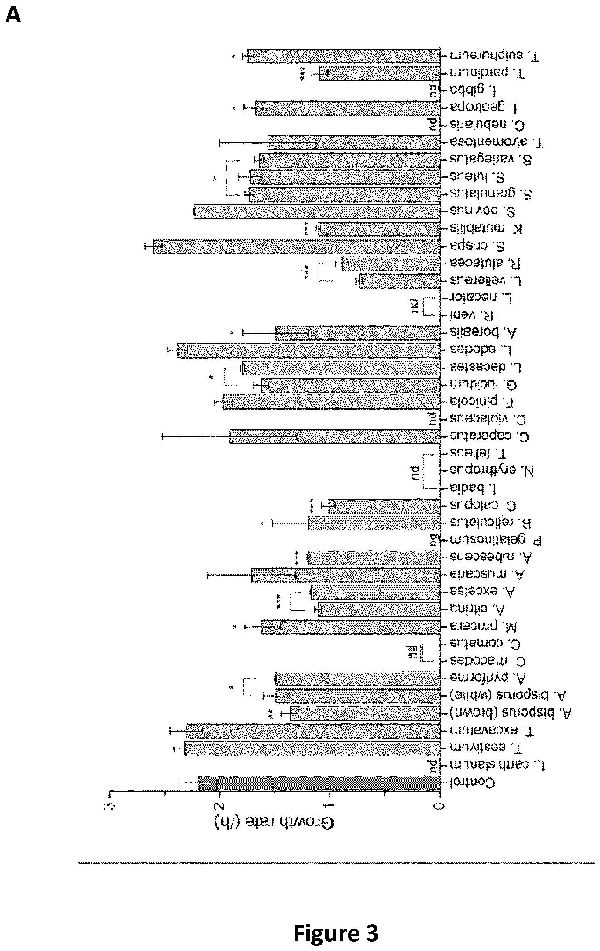

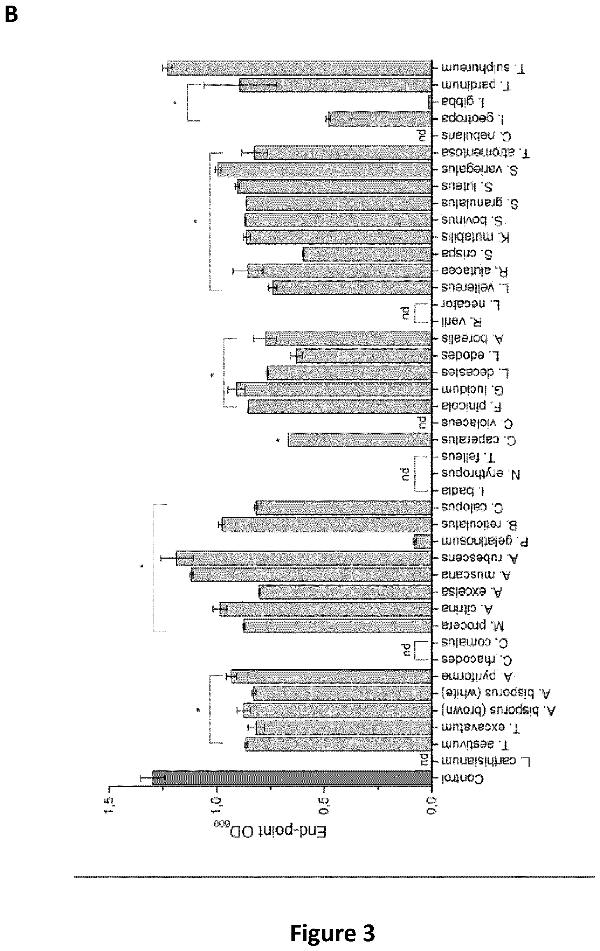

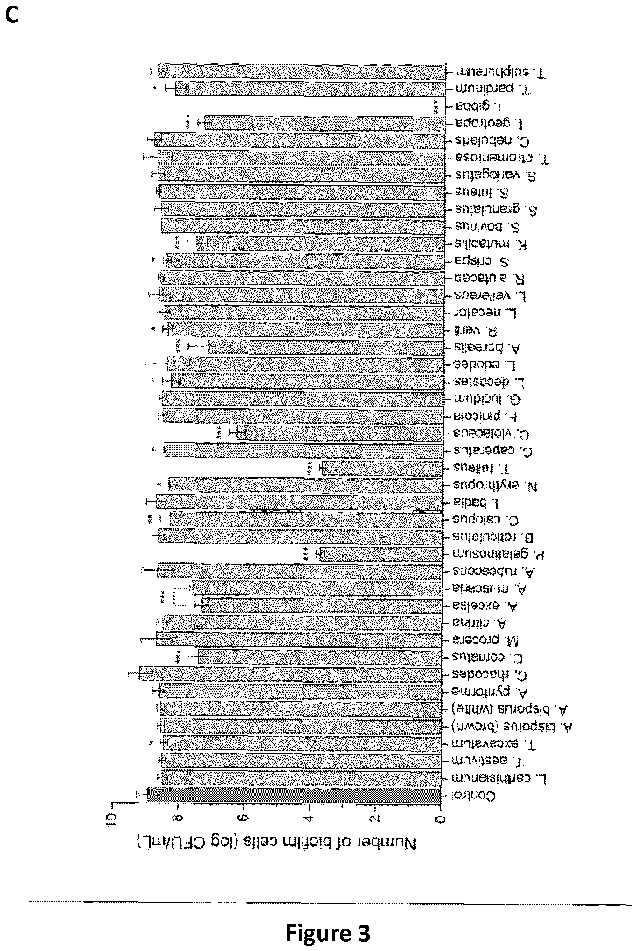

**Figure 3****.** Influence of the 42 fungal extracts at relative concentration 50.0% (1:1 dilution) on *Salmonella* Infantis ŽM9 growth rate (A), end-point optical density (B) and number of biofilm cells (C). Data are means ± standard deviation. (A, C) *, *p* <0.05; **, *p* <0.01; ***, *p* <0.001; <control (Dunnett's t-tests). (B) *, *p* <0.05 *versus* control (Mann-Whitney U tests). Horizontal lines: significance applies to all columns covered. nd, not determined; ng, no growth.

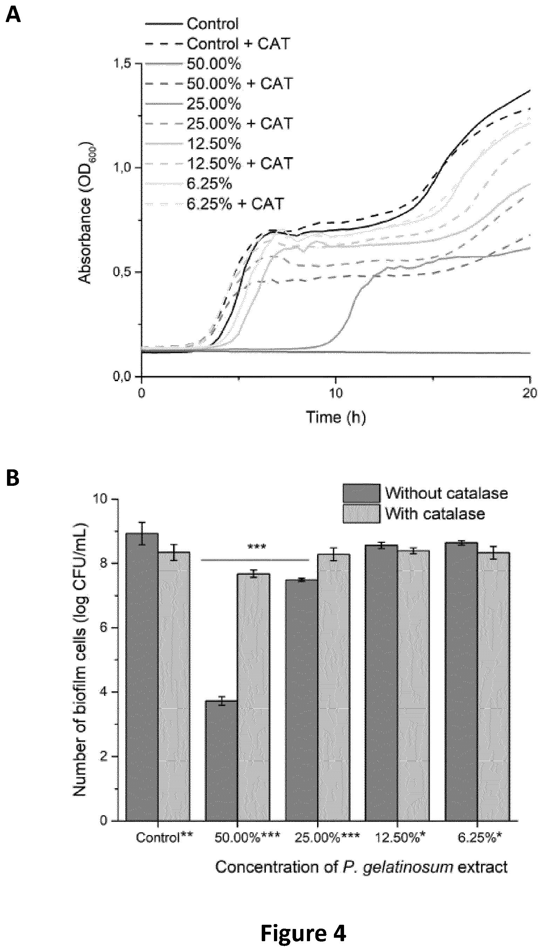

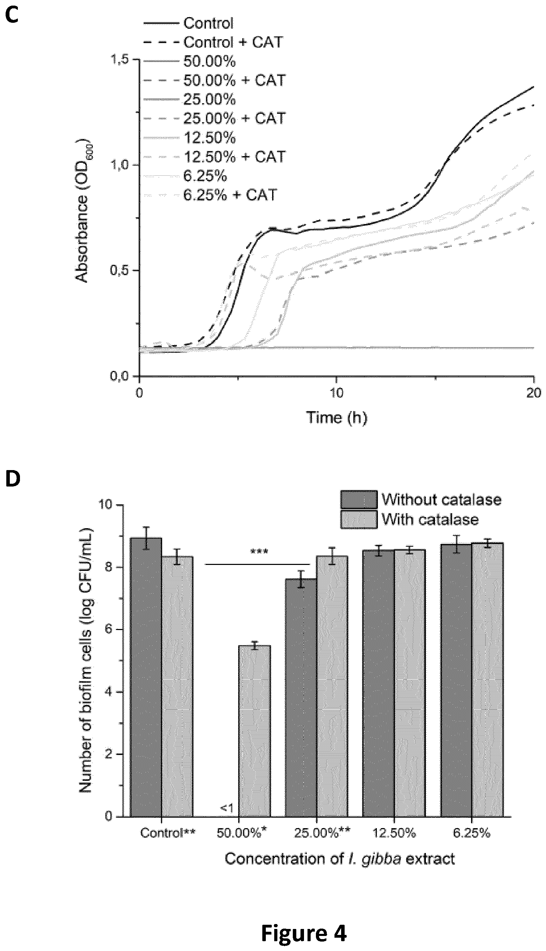

**Figure 4****.** Influence of *Pseudohydnum gelatinosum* (A, B) and *Infundibulicybe gibba* (C, D) extracts on *Salmonella* Infantis ŽM9 growth (A, C) and number of biofilm cells (B, D). (C) Relative concentrations for serial dilutions of extracts as indicated (50.0%, 1:1; 25.0%, 1:4; 12.50%, 1:8; 6.25%, 1:16), without and with catalase (CAT). (B, D) Data are means ± standard deviation. Columns: ***, *p* <0.001 (without or with catalase) <control (Dunnett's t-tests). X axis: *, *p* <0.05; **, *p* <0.01; ***, *p* <0.001; (within fractions) with *versus* without catalase (t-tests). Horizontal lines: significance applies to all columns covered.

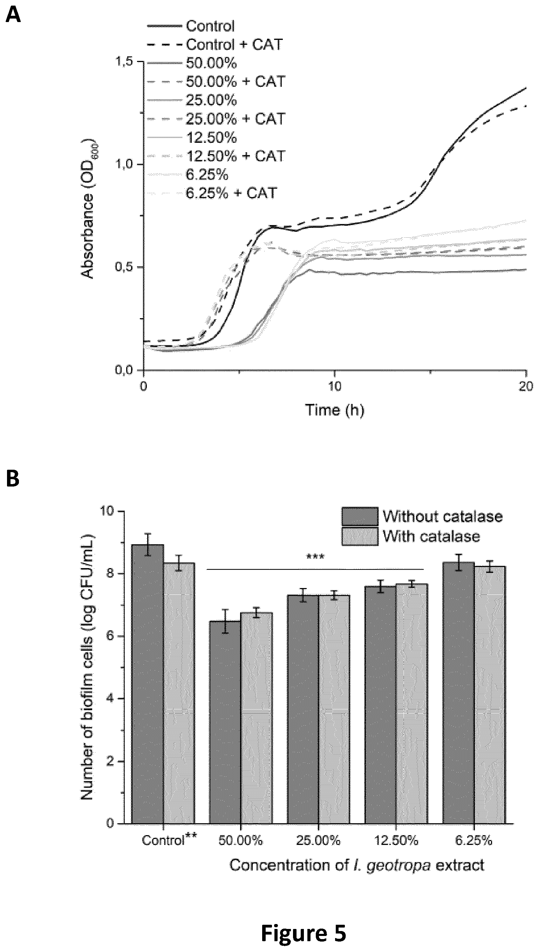

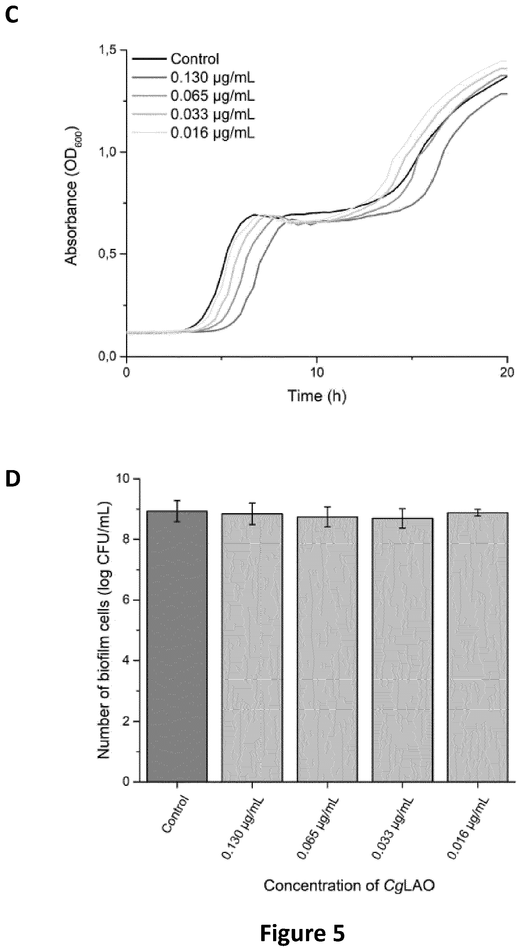

**Figure 5****.** (A, B) Influence of *Infundibulicybe geotropo* extract on *Salmonella* Infantis ŽM9 growth (A) and number of biofilm cells (B). Relative concentrations for serial dilutions of extracts as indicated (50.0%, 1:1; 25.0%, 1:4; 12.50%, 1:8; 6.25%, 1:16), without and with catalase (CAT). Columns: *** *p* <0.001, (without or with catalase) <control (Dunnett's t-tests). (horizontal line: significance applies to all columns covered). X axis: ** *p* <0.01 (within fractions) with *versus* without catalase (t-tests). (C, D) Influence of CgLAO on *Salmonella* Infantis ŽM9 growth (C) and number of biofilm cells (D), for serial dilutions as indicated. (B, D) Data are means ± standard deviation.

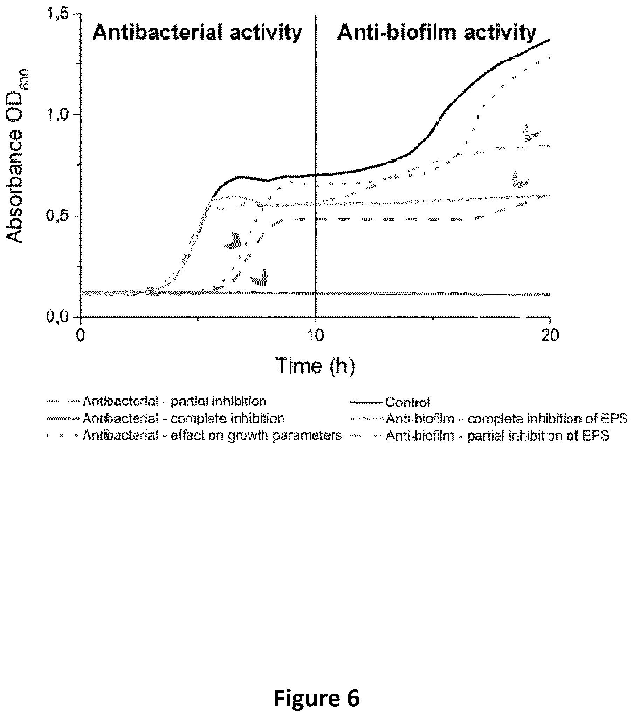

**Figure 6****.** Growth curve analysis of *Salmonella* Infantis ŽM9 to selectively define antibacterial and anti-biofilm activities. Arrowheads: dark grey, inhibition of growth; light grey, inhibition of biofilm formation.

The present invention is now described in more detail below.

### Detailed description of the invention

The present invention is based on the finding that spectrophotometric detection of the growth of bacteria and other microorganisms can be used to distinguish between growth-inhibiting and biofilm-inhibiting activities of bioactive compositions and compounds. Particularly, the present inventors have noticed a surprising secondary increase in the growth curve. This was confirmed to be a consequence of the formation of biofilm biomass, which is probably due to EPS production. By defining the microbial growth according to the growth curve, it is thus possible to evaluate whether tested compositions or compounds show antimicrobial and/or anti-biofilm activities - antimicrobial in terms of an effect on growth, and anti-biofilm in terms of an effect on production of biofilm biomass. Although antimicrobial compositions and compounds can reduce biofilm production, it is important to distinguish between these two concepts. The use of growth curve analysis thus allows one to simultaneously determine antimicrobial and/or anti-biofilm activities.

The present invention provides a fast and simple approach to distinguish between antimicrobial and anti-biofilm activities in a high-throughput setting, such that it can be easily implemented in screening and in further bioassay-based purification of novel alternatives to antibiotics.

The present invention thus provides in a first aspect a method for identifying a composition or compound having antimicrobial and/or anti-biofilm activities against a microorganism of interest, comprising the steps of a) providing a composition or compound, b) bringing the composition or compound in contact with a microorganism of interest in a growth medium; c) incubating the microorganism in the presence of the composition or compound; d) determining the growth of the microorganism by measuring the optical density in intervals during the incubation under c); e) preparing a growth curve from the measurement obtained under d); and f) analyzing the growth curve to determine if the composition or compound has antimicrobial and/or anti-biofilm activities against the microorganism;
wherein step f) comprises comparing the growth curve prepared in step e) with a control growth curve which is obtained in accordance with steps c) to e) with all conditions being identical except that the microorganism is incubated in the absence of the composition or compound;
wherein the control growth curve exhibits a first phase including an increase in optical density within a first period of time, a second phase including an increase in optical density within a second period of time, and a stationary phase between the first phase and the second phase.

The composition or compound may be brought in contact with the microorganism in step b) by mixing an inoculum of the microorganism in growth medium with the composition or compound. The final volume of the mixture may be selected based on the lab ware, e.g., test tube, used and the technical limitations of the instrument, e.g., the spectrometer or densitometer, with which the optical density is measured. Generally, it is expected that the inoculum of the microorganism in growth medium can be mixed with the composition or compound with a final volume of up to 10 ml. According to some embodiments, the inoculum of the microorganism in growth medium is mixed with the composition or compound with a final volume of up to 1 ml. According to some embodiments, the inoculum of the microorganism in growth medium is mixed with the composition or compound with a final volume of 1 µ! to 10 ml. According to some embodiments, the inoculum of the microorganism in growth medium is mixed with the composition or compound with a final volume of 1 µl to 1 ml. According to some embodiments, the inoculum of the microorganism in growth medium is mixed with the composition or compound with a final volume of 10 µl to 1 ml. According to some embodiments, the inoculum of the microorganism in growth medium is mixed with the composition or compound with a final volume of 50 to 500 µL. According to some embodiments, the inoculum of the microorganism in growth medium is mixed with the composition or compound with a final volume of 100 to 300 µl. According to some embodiments, the final volume is 100 µL. According to some embodiments, the final volume is 200 µL.

The concentration of the inoculum of the microorganism may be any suitable concentration, such as a concentration in a range from 10¹ to 10⁹ CFU/mL. According to some embodiments, the inoculum of the microorganism has a concentration in a range from 10² to 10⁸ CFU/mL. According to some embodiments, the inoculum of the microorganism has a concentration in a range from 10³ to 10⁷ CFU/mL. According to some embodiments, the inoculum of the microorganism has a concentration in a range from 10⁴ to 10⁶ CFU/mL. According to some embodiments, the inoculum of the microorganism has a concentration of 10⁵ CFU/mL.

The composition or compound may be mixed with the inoculum in any suitable amount/concentration and any suitable ratio. The amount/concentration and ratio may vary depending on the actual nature of the composition or compound, and can be determined by the skilled person. By way of non-limiting example, if the composition is a natural extract, such as an extract or a fraction thereof derived from a fungus, the extract or fraction may be used directly or in form of a x-fold dilution, e.g., a dilution ranging from 1:1 to 1:128, such as a dilution ranging from 1:1 to 1:16, and may be mixed with the inoculum in a ratio ranging from 10:1 v/v to 1:10 v/v, such as in a ratio of 1:1 v/v.

The incubation in step c) may be carried out for any suitable period of time, for example, for a period from 5 to 120 hours. Generally, a suitable period of time is one which permits the growth of the microorganism of interest under control conditions, i.e. when incubated in the absence of the composition or compound). The period of time may thus vary depending on the actual microorganism employed in the method, and can be determined by the skilled person. According to some embodiments, the incubation in step c) is carried out for a period from 10 to 60 hours. According to some embodiments, the incubation in step c) is carried out for a period from 10 to 50 hours. According to some embodiments, the incubation in step c) is carried out for a period from 15 to 40 hours. According to some embodiments, the incubation in step c) is carried out for a period from 15 to 25 hours. According to some embodiments, the incubation in step c) is carried out for a period from 17 to 22 hours. According to some embodiments, the incubation in step c) is carried out for 20 hours.

The incubation in step c) may be carried out at any suitable temperature, such as at a temperature from 4°C to 70 °C. Generally, the incubation temperature is one which permits the growth of the microorganism of interest under control conditions, i.e. when incubated in the absence of the composition or compound. The temperature may thus vary depending on the actual microorganism employed in the method, and can be determined by the skilled person. Thus, according to some embodiments, the incubation in step c) is carried out at temperature 4°C to 70 °C. According to some embodiments, the incubation in step c) is carried out at a temperature from 4°C to 60 °C. According to some embodiments, the incubation in step c) is carried out at a temperature from 4° to 50 °C. According to some embodiments, the incubation in step c) is carried out at a temperature from 4° to 47 °C. According to some embodiments, the incubation in step c) is carried out at a temperature from 20 to 47 °C. According to some embodiments, the incubation in step c) is carried out at a temperature from room temperature to 40 °C. According to some embodiments, the incubation in step c) is carried out at a temperature from 30 to 40 °C. According to some embodiments, the incubation in step c) is carried out at a temperature from 35 to 38 °C. According to some embodiments, the incubation in step c) is carried out at 37 °C.

According to some embodiments, the incubation in step c) is carried out for 20 hours at 37 °C.

The growth medium employed may be any conventional medium suitable for culturing the microorganism in question, and may be composed according to the principles of the prior art. The medium will usually contain all nutrients necessary for the growth and survival of the respective microorganism, such as carbon and nitrogen sources and other inorganic salts. Suitable media, e.g. minimal or complex media, are available from commercial suppliers, or may be prepared according to published receipts, e.g. the American Type Culture Collection (ATCC) Catalogue of strains. Non-limiting standard medium well known to the skilled person include Luria Bertani (LB) broth, Sabouraud Dextrose (SD) broth, MS broth, Yeast Peptone Dextrose, BMMY, GMMY, or Yeast Malt Extract (YM) broth, which are all commercially available. A non-limiting example of suitable media for culturing bacterial cells include minimal media and rich media such as Luria Broth (LB), M9 media, M17 media, SA media, MOPS media, Terrific Broth, YT and others. Suitable media for culturing eukaryotic cells, such as yeast cells, are YPD, YNB or malt extract. The medium for culturing eukaryotic cells, such as yeast cells, may also be any kind of minimal media such as Yeast minimal media.

The optical density in step d) may be measured at any suitable interval. Generally, a suitable interval is one which allows the preparation of a growth curve (e.g., with time on the abscissa axis and the measured optical density on the ordinate axis). A suitable interval can be determined by the skilled person, but generally may range from 10 seconds to 120 minutes. Thus, according to some embodiments, the optical density in step d) is measured in intervals of 10 seconds to 120 minutes. According to some embodiments, the optical density in step d) is measured in intervals of 10 seconds to 60 minutes. According to some embodiments, the optical density in step d) is measured in intervals of 30 seconds to 60 minutes. According to some embodiments, the optical density in step d) is measured in intervals of 1 to 60 minutes. According to some embodiments, the optical density in step d) is measured in intervals of 1 to 45 minutes. According to some embodiments, the optical density in step d) is measured in intervals of 10 to 30 minutes. According to some embodiments, the optical density in step d) is measured in intervals every 1, 2, 5, 10, 15, 20, 25, 30, 35, 40, 45, 50, 55 or 60 minutes. According to some embodiments, the optical density in step d) is measured in intervals every 10, 15, 20 or 30 minutes. According to some embodiments, the optical density in step d) is measured in intervals every 30 minutes.

Steps b) and c) may be carried out in any suitable reaction vessel. Generally, a suitable reaction vessel is one which allows the incubation of the microorganism in the presence of the composition or compound, while at the same time permits a measurement of the optical density. The actual nature of the reaction vessel may vary and will depend on the technical limitations of the instrument, e.g., the spectrometer or densitometer, with which the optical density is measured. Non-limiting examples of suitable reaction vessels are test tubes or flasks. According to some embodiments, steps b) and c) are carried out in a test tube or flask. According to some embodiments, steps b) and c) are carried out in a test tube, such as a well of a microtiter plate. According to some embodiments, steps b) and c) are carried out in a well of a microtiter plate, such as a well in a microtiter plates with 96 wells.

The optical density may be measured using, for example, a spectrometer or densitometer. Suitable spectrometers or densitometers are known to the skilled person.

According to some embodiments, the optical density is measured using a spectrometer, such as a UV spectrometer or a UV-VIS spectrometer. According to some embodiments, optical density is measured using a UV spectrometer. Generally, the optical density in step d) may be measured at a wavelength ranging from 500 to 700 nm. According to some embodiments, the optical density in step d) is measured at wavelength from 550 to 650 nm. According to some embodiments, the optical density in step d) is measured at wavelength of 600 nm.

As noted above, the use of growth curve analysis allows one to simultaneously determine antimicrobial and anti-biofilm activities. Thus, by defining the microbial growth according to the growth curve, it is thus possible to evaluate whether tested compositions or compounds show antimicrobial and/or anti-biofilm activities. A growth curve for subsequent analysis in accordance with the invention will be prepared from the optical density measurement, for example, with time on the abscissa axis and the measurement obtained under d) on the ordinate axis.

Analyzing the growth curve in step f) to determine if the composition or compound has antimicrobial and/or anti-biofilm activities against the microorganism may comprise comparing the growth curve prepared in step e) with a control growth curve. The control growth curve may be obtained in accordance with steps c) to e) with all conditions being identical except that the microorganism is incubated in the absence of the composition or compound. Generally, the control growth curve will exhibit a first phase including an increase in optical density within a first period of time and a second phase including an increase in optical density within a second period of time. As indicated above, the increase in optical density within the first phase is attributed to the growth of the microorganism, while the increase in optical density within the second phase is the consequence of the formation of biofilm biomass by the microorganism.

The first period of time is usually selected to cover the lag phase, the exponential growth phase and the transition to stationary growth phase. According to some embodiments, the first period of time corresponds to the first half of the incubation time under c) and the second period corresponds to the second half of the incubation time under c). According to some embodiments, the first period of time corresponds to the first 10 hours of the incubation time under c) and the second period corresponds to the remaining incubation time.

Suitably, the control growth curve further exhibits a stationary phase between the first phase and the second phase, that is a phase during which the optical density reaches a state where there is no net increase, i.e. where it reaches a plateau.

Once a control growth curve with the above characteristic has been established for the microorganism of interest, this allows a comparison with the growth curve prepared for the microorganism of interest when incubated in the presence of the composition or compound tested.

In this respect, the composition or compound is considered having antimicrobial activity against the microorganism if the growth curve, when compared to the control growth curve, shows a decrease in the optical density in the first phase within the first period of time. For example, the composition or compound may be considered having antimicrobial activity against the microorganism if the growth curve, when compared to the control growth curve, shows a decrease of at least 10%, at least 20%, at least 30%, at least 40%, at least 50%, at least 60%, at least 70%, at least 80%, at least 90% or at least 100% in the optical density in the first phase within the first period of time.

Further, the composition or compound is considered having anti-biofilm activity against the microorganism if the growth curve, when compared to the control growth curve, shows a decrease in the optical density in the second phase within the second period of time. For example, the composition or compound is considered having anti-biofilm activity against the microorganism if the growth curve, when compared to the control growth curve, shows a decrease of at least 10%, at least 20%, at least 30%, at least 40%, at least 50%, at least 60%, at least 70%, at least 80%, at least 90% or at least 100% in the optical density in the second phase within the second period of time.

Alternatively, or in addition, analyzing the growth curve in step f) to determine if the composition or compound has antimicrobial and/or anti-biofilm activities against the microorganism may comprise evaluating at least one growth parameter for the first phase. Such evaluation provides quantitative assessment and therefore enables knowledgeable comparison of the efficacy of the antimicrobial activity. According to some embodiments, step f) comprises evaluating at least one growth parameters for the first phase. According to some embodiments, step f) comprises evaluating at least two growth parameters for the first phase. According to some embodiments, step f) comprises evaluating at least three growth parameters for the first phase. According to some embodiments, step f) comprises evaluating at least four growth parameters for the first phase.

Suitable growth parameters are, for example, lag phase length, carrying capacity, growth rate, doubling time, time in which the population density reaches half of the carrying capacity, and end-point optical density (OD). "Lag phase length" is the time of adaptation of microorganism to environment before starting exponential growth. "Carrying capacity" represents the maximum population size of microorganism a particular environment can support. "Growth rate" is a change in number of individuals in a population over time. "Doubling time" is the time interval required for microorganisms to divide.

If at least one parameter selected from the group consisting of carrying capacity, growth rate, and end-point optical density (OD) is included in the evaluation, the composition or compound is considered having antimicrobial activity against the microorganism if the at least one parameter in the growth curve is lower than the corresponding parameter in the control growth curve. More specifically, if at least one parameter selected from the group consisting of carrying capacity, growth rate, and end-point optical density (OD) is included in the evaluation, the composition or compound is considered having antimicrobial activity against the microorganism if the at least one parameter is decrease by at least 10%, at least 20%, at least 30%, at least 40%, at least 50%, at least 60%, at least 70%, at least 80%, at least 90% or at least 100% compared to the corresponding parameter in the control growth curve

Alternatively, or in addition, if at least one parameter selected from the group consisting of the lag phase length, doubling time, and time in which the population density reaches half of the carrying capacity is included in the evaluation, the composition or compound is considered having antimicrobial activity against the microorganism if the at least one parameter is longer than the corresponding parameter in the control growth curve. More specifically, if at least one parameter selected from the group consisting of the lag phase length, doubling time, and time in which the population density reaches half of the carrying capacity is included in the evaluation, the composition or compound is considered having antimicrobial activity against the microorganism if the at least one parameter is increased by at least 10%, at least 20%, at least 30%, at least 40%, at least 50%, at least 60%, at least 70%, at least 80%, at least 90% or at least 100% compared to the corresponding parameter in the control growth curve.

Analyzing the growth curve in step f) may also comprise evaluating the end-point optical density (OD) for the second phase.

Based on such parameter evaluation, the composition or compound is considered having anti-biofilm activity against the microorganism if the end-point OD in the growth curve is lower than the end-point OD in the control growth curve.

Optionally, analyzing the growth curve in step f) may also comprise a statistical analysis of the growth curve. Such analysis allows objective evaluation of the analyzed activities. Various statistical analysis methods are known to the skilled person, and can be employed in accordance with the present invention, such as tests for evaluation of data normality and homogeneity of variance, based on which further parametric or non-parametric tests can be selected. By way of non-limiting example, a statistical analysis of the growth curve may comprise:
i) testing of assumption of normality (i.e. Shapiro Wilk test) and assumption of homogeneity of variance (i.e. Levene test);
ii) subsequently, if both are met, analysis of variance with post-hoc Dunett t test (comparison with control) or Tukey's test;
iii) subsequently, if there is no homogeneity of variance, a Welch analysis of variance with post-hoc Games-Howell test;
iv) subsequently, if the results do not meet assumptions of normality and homogeneity of variance, non-parametric Mann-Whitney U test to compare each value with the control; and
v) showing the correlation between the end-point values of the growth curve, number of biofilm cells and biofilm formation as determined by crystal violet by calculating the Pearson correlation.

The microorganism of interest may be any single-celled or multicellular microorganism, including archaea, bacteria, fungi, and protists. Preferably, the microorganism of interest is a bacterium or yeast.

According to some embodiments, the microorganism of interest is a bacterium, which may be a Gram-positive or Gram-negative bacterium. According to some embodiments, microorganism of interest is a gram-negative bacterium.

According to some embodiments, the microorganism of interest is a bacterium selected from the group consisting of a food-borne pathogenic bacteria, medically important bacteria including clinically important bacteria, bacteria important to dental health, bacteria important to veterinary medicine and environmentally important bacteria.

According to some embodiments, microorganism of interest is an enteropathogenic and/or food-borne pathogenic bacterium.

According to some embodiments, microorganism of interest is a bacterium of the family *Enterobacteriaceae.*

According to some embodiments, microorganism of interest is a bacterium of the genus *Listeria, Escherichia, Yersinia, Vibrio, Salmonella, Pseudomonas, Staphylococcus, Streptococcus, Bacillus, Aeromonas, Campylobacter* or *Shigella.*

According to some embodiments, microorganism of interest is a bacterium of the genus Salmonella.

According to some embodiments, microorganism of interest is a bacterium selected from the group consisting of: *Listeria monocytogenes, Listeria innocua, Yersinia enterocolitica, Vibrio parachaemoliticus, Escherichia coli, Salmonella enterica,* including *Salmonella* Enteritidis and *Salmonella enterica* subps. *enterica* serovar Infantis *(Salmonella* Infantis), *Staphylococcus cures, Staphylococcus epidermidis, Streptococcus pyogenes, Pseudomonas aeruginosa, Pseudomonas fluorescens, Bacillus subtilis, Bacillus cereus, Aeromonas hydrophila, Aeromonas salmonicida, Campylobacter jejuni, Campylobacter coli* and *Shigella dysenteriae.*

According to some embodiments, microorganism of interest is *Salmonella* Infantis.

According to some embodiments, microorganism of interest is a yeast.

According to some embodiments, microorganism of interest is a yeast of the genus *Candida, Cryptococcus, Pichia* or *Saccharomyces.*

According to some embodiments, microorganism of interest is a yeast selected from the group consisting of: *Candida albicans, Candida auris, Candida glabrata, Candida parapsilosis, Candida tropicalis, Candida krusei, and Cryptococcus neoformans.*

The composition or compound to be tested in accordance with the invention may be any composition or compound for which there is an interest in determining whether such compositions or compounds has antimicrobial and/or anti-biofilm activities.

The composition may, for example, be a composition derived from a natural source, such as an environmental sample, such as soil or seabed. More specifically, the composition may, for example, be a natural extract or fraction thereof, such as an extract or a fraction thereof derived from a fungus, plant, algae, archaea, bacterium or animal cell or tissue. The composition may also be a composition comprising one or more chemically synthesized compounds.

Likewise, the compound may be a compound which has been isolated from a natural source, such as an environmental sample, such as soil or seabed. More specifically, the compound may, for example, be a compound which has been isolated from a natural extract or fraction thereof, such as from an extract or fraction thereof derived from a fungus, plant, algae, archaea, bacterium or animal cell or tissue. The compound may also be a chemically synthesized compound.

The present disclosure thus provides in a further aspect the use of a composition or compound identified by carrying out the method of the invention for inhibiting, reducing or preventing the growth of a microorganism as defined herein.

The present disclosure thus provides in a further aspect the use of a composition or compound identified by carrying out the method of the invention for inhibiting, reducing or preventing biofilm formation of a microorganism as defined herein.

Having generally described this invention, a further understanding can be obtained by reference to certain specific examples, which are provided herein for purposes of illustration only, and are not intended to be limiting unless otherwise specified.

### Examples

### Methods

### Fungal extracts

Cold aqueous fungal extracts were prepared from fruiting bodies of 38 wild fungi collected in Slovenia and from five cultivated fungi (Table 4). The taxonomic classification follows the Index Fungorum database (http://www.indexfungorum.org). The extracts were prepared as described in [23]. Dry weights of the extracts were determined by oven drying to constant weight at 130 °C. These crude cold aqueous extracts were stored in aliquots at -20 °C. The antimicrobial and anti-biofilm activities of the extracts were tested at 1:1 dilution (relative concentration 50%) (v/v) in tryptic soy broth (Biolife, Milan, Italy). For the active extracts at this concentration, additional 2-fold dilutions were tested; i.e., 1:4, 1:8 and 1:16 (relative concentrations 25.00%, 12.50% and 6.25%, respectively).

### Bacteria and growth conditions

The *Salmonella enterica* subps. *enterica* serovars included were: Infantis ŽM9 (isolated from chicken meat) and ŽM350 (isolated from eggs); Enteritidis ŽM351 (isolated from egg melange); Typhimurium ŽM352 (isolated from turkey meat) and ŽM375 (ATCC14028); and Hadar ŽM378 (isolated from poultry); all of these were obtained from the culture collection of the Laboratory for Food Microbiology at the Department of Food Science, Biotechnical Faculty, University of Ljubljana, Slovenia. The bacteria were stored at -80 °C in tryptic soy broth with 15% glycerol (Kemika, Zagreb, Croatia), revitalised on tryptic soy agar (Biolife, Milan, Italy), and incubated for 24 h at 37 °C (SP109 incubator; Kambič, Semič, Slovenija). Standardised inocula were prepared in tryptic soy broth by diluting cultures to 5 log CFU/mL, which were used for all analyses.

### Growth kinetics of Salmonella as influenced by fungal extracts

To determine the effects of the extracts on growth kinetics, the growth of *Salmonella* spp. was evaluated by direct measurements of optical density. First, the effects of the *Pleurotus ostreatus* extract on six *S. enterica* serovars were evaluated, followed by the effects of 42 fungal extracts on S. Infantis ŽM9. The standardised inoculum and fungal extracts were mixed in each well of 96-well microtiter plates (Thermo Scientific Nunc, Rochester, NY, USA), to a final volume of 200 µL (1:1, v/v), which were then incubated in a microtiter plate reader (Varioskan Lux; Thermo Fischer Scientific, Waltham, MA, US) at 37 °C, with kinetic measurements of absorbance at 600 nm taken every 20 min (61 measurements in total). For extracts where the initial optical density (OD₆₀₀) was above 0.500, the data were not analysed because the color of the extracts changed the shapes of the growth curves.

Due to the specific changes in the growth curves showing a second phase of optical density increase after transition to stationary phase (the secondary increase), only the data from 0 to 12 h were used to obtain the following growth parameters: (i) lag time, as an adaptation to the growth conditions; (ii) carrying capacity, as the maximum possible population size within the first 12 h; (iii) intrinsic growth rate within the first 12 h; (iv) time in which the population density reached half of the carrying capacity; and (v) doubling time within the first 12 h. These parameters were obtained using the R package Growthcurver [24]. The lag time was determined using the DMFit application [25]. The end-point OD₆₀₀ was evaluated after 20 h.

### Anti-biofilm activities of fungal extracts

The anti-biofilm activities of the fungal extracts were evaluated as the number of biofilm cells and biofilm biomass after 20 h. Microtiter plates were prepared as described for the growth kinetics, then incubated for 20 h at 37 °C, and washed three times with saline solution (0.9% sodium chloride; Merck, Darmstadt, Germany). The number of biofilm cells was determined for all of the extracts and the biofilm biomass was determined for the *P. ostreatus* extract only. To determine viable biofilm cells, 200 µL saline was added to each well, with sonication to detach the cells. These biofilm cell suspensions were then diluted and spot plated onto tryptic soy agar, incubated at 37 °C for 20 h to 24 h, counted and expressed as log CFU/mL, as means ± standard deviation, as previously described in [18]. A crystal violet assay was used to determine the biofilm biomass. After washing, the plates were dried and then dyed with 0.1% crystal violet (Merck, Darmstadt, Germany), and finally the absorbance at 584 nm was determined, as previously described in [18]. The negative control was deducted from the absorbances, and the biofilm inhibition (%) was then calculated, as means ± standard deviation.

### Statistical analysis

The data are expressed as means ± standard deviation. Assumption of normality (Shapiro Wilk) and assumption of homogeneity of variance (Levene) were tested. If both were met, that data were analysed using analysis of variance with *post-hoc* Dunett t-tests *(versus* control) or Tukey's tests. If only two groups were compared, t-test was used. If there was no homogeneity of variance, Welch analysis of variance with *post-hoc* Games-Howell tests were performed. If the data did not meet assumptions of normality and homogeneity of variance, non-parametric Mann-Whitney U tests were used to compare each value with the control. The different statistical tests used are indicated where relevant. To show correlations between end-point values of the growth curves, numbers of biofilm cells and biofilm formation as determined by crystal violet, Pearson correlations were calculated. For all of the analyses, *p* <0.05 was considered as statistically significant. Statistical analyses were performed using SPSS Statistics V23 (IBM Corporation, North Castle, NY, USA).

### Results

### Modulation of growth and biofilm formation of Salmonella enterica serovars by the Pleurotus ostreatus extract

The kinetics of the growth curves of six *Salmonella enterica* serovars were determined initially. Interestingly, after the onset of the stationary phase, a marked increase in the growth curves was observed, which was most pronounced in two S. Infantis strains (Figure 1, black lines; Table 2). Differences were also seen for biofilm formation of six *Salmonella enterica* serovars, with the number of biofilm cells of the S. Infantis strains ^{~}1 log CFU/mL greater than for the other strains tested (Table 1).

We then determined the effects on the growth kinetics of all of the tested *Salmonella* strains of a *P. ostreatus* extract at 1:1 and 1:16 dilutions, representing 50.0% and 6.25% relative concentrations from the undiluted *P. ostreatus* extract (Figure 1, Table 2). Strain-specific effects were also seen here. Addition of the extract at these two different dilutions showed concentration dependence. This was particularly apparent for the carrying capacity, which defines the maximum possible population size under the given conditions, here defined for 12 h of bacterial growth, and measured as the absorption at 600 nm (A₆₀₀). This carrying capacity reached higher values at the greater extract relative concentrations of 6.25% than at 50.0%. However, greater significant differences were seen for the end-point OD₆₀₀, as defined at 20 h of bacterial growth (Table 2), and the shapes of the growth curves (Figure 1). Interestingly, addition of the *Pleurotus* extract at lower and higher relative concentration prevented the secondary increase in the growth curve, which was most pronounced in S. Infantis. In addition, the extract significantly reduced the number of biofilm cells of all of the tested strains, with reductions by up to a factor of 2 log units for S. Infantis, and up to 1 log unit for the other *Salmonella* serovars (Table 1). The effects on the decrease in the numbers of biofilm cells was also concentration dependent, in terms of the *Pleurotus* extract dilutions.

We were particularly interested here in the secondary increase in the growth curves of S. Infantis (Figure 1). Assuming that this increase in the stationary phase is due to the formation of EPS, and that the *Pleurotus* extract inhibits this, we further tested seven 2-fold dilutions of this extract (from 1:2 [50.0%] to 1:128 [0.78%]). In addition to the end-point OD₆₀₀ and the number of biofilm cells, the biofilm biomass was determined using a crystal violet assay. These data are shown in Figure 2, where the *Pleurotus* extract strongly inhibited the formation of biofilm biomass in S. Infantis in a concentration-dependent manner. With no inhibition seen at the highest dilutions (≥1:32; ≤3.13%), the inhibition became significant at the 1:16 (6.25%) dilution, at 33.8% ±5.5%. At these lower dilutions of the *Pleurotus* extract, the number of biofilm cells was also significantly reduced - thus, the number of biofilm cells was significantly reduced at the same *Pleurotus* extract dilutions where there were significant reductions in the end-point OD and the biofilm biomass (Figure 2, ≤1:16 [>6.25%]).

In addition, Pearson correlation coefficients were calculated (Table 3), which showed strong correlations between these three parameters of end-point OD₆₀₀, crystal violet absorbance (i.e., biofilm biomass), and number of biofilm cells. These data also indicated that the secondary increase in the growth curves was associated with the formation of biofilm biomass. With this knowledge, screening was also performed for 42 additional fungal aqueous extracts in terms of their bioactivities against S. Infantis ŽM9.

**Table 1. Number of biofilm cells of Salmonella enterica subps. enterica serovars affected by Pleurotus ostreatus extract (POE) at relative concentrations of 50% (dilution 1:1) and 6.25% (dilution 1:16). Data are means ± standard deviation.**

| **Strain** | **Number of biofilm cells (log CFU/mL)** | | |
|---|---|---|---|
| | **Control** | **POE 50.0%** | **POE 6.25%** |
| S. Infantins ŽM9 | 8.93 ± 0.35^{a} | 6.43 ± 0.29^{b} | 7.83 ± 0.41^{c} |
| S. infantis ŽM350 | 8.67 ± 0.17^{a} | 6.71 ± 0.72^{b} | 7.84 ± 0.86^{c} |
| S. Enteritidis ŽM351 | 7.73 ± 0.27^{a} | 6.35 ± 0.43^{a} | 7.48 ± 0.18^{a} |
| S. Typhimurium ŽM352 | 7.13 ± 0.43^{a} | 6.34 ± 0.23^{b} | 6.83 ± 0.84^{a} |
| S. Typhimurium ŽM375 | 7.70 ± 0.40^{a} | 6.91 ± 0.59^{b} | 7.10 ± 0.48^{b} |
| S. Hadar ŽM378 | 7.74 ± 0.14^{a} | 6.58 ± 0.47^{b} | 7.74 ± 0.22^{a} |

| | | | |
|---|---|---|---|
| Different superscript lower case letters across rows are significantly different (p <0.05; Tukey's tests) | | | |

**Table 2. Growth parameters and end-point optical density (OD₆₀₀) of the Salmonella enterica subps. enterica serovars as influenced by the Pleurotus ostreatus extract at relative concentrations of 50.0% (dilution 1:1) and 6.25% (dilution 1:16). Data are means ± standard deviation.**

| **Strain** | ***Pleurotus ostreatus* extract dilution** | **Growth parameters** | | | | | **End-point (OD₆₀₀)** |
|---|---|---|---|---|---|---|---|
| | | **Lag (h)** | **Carrying capacity (A₆₀₀)** | **Growth rate (/h)** | **Time to reach 50% carrying capacity (h)** | **Doubling time (h)** | |
| ***S*. Infantis ŽM9** | **Control** | 3.60 ± 0.17 | 0.588 ± 0.017 | 2.19 ± 0.17 | 4.65 ± 0.11 | 0.33 ± 0.03 | 1.298 ± 0.055 |
| | **50.0%** | 4.07 ± 0.04* | 0.579 ± 0.016 | 3.13 ± 0.20** | 4.80 ± 0.01 | 0.22 ± 0.01* | 0.623 ± 0.028*** |
| | **6.25%** | 4.14 ± 0.01** | 0.642 ± 0.009*** | 2.65 ± 0.01** | 4.98 ± 0.02** | 0.26 ± 0.01* | 0.711 ± 0.003*** |
| ***S*. Infantis ŽM350** | **Control** | 4.37 ± 0.01 | 0.619 ± 0.004 | 2.54 ± 0.09 | 5.25 ± 0.03 | 0.27 ± 0.01 | 1.323 ± 0.006 |
| | **50.0%** | 4.06 ± 0.01*** | 0.656 ± 0.008** | 2.92 ± 0.16* | 4.82 ± 0.03*** | 0.24 ± 0.01* | 0.717 ± 0.013*** |
| | **6.25%** | 4.09 ± 0.03*** | 0.708 ± 0.001*** | 2.28 ± 0.01 | 5.06 ± 0.02** | 0.30 ± 0.01* | 0.832 ± 0.006*** |
| ***S*. Enteritidis ŽM351** | **Control** | 3.35 ± 0.01 | 0.711 ± 0.006 | 1.42 ± 0.02 | 4.92 ± 0.02 | 0.49 ± 0.01 | 1.074 ± 0.011 |
| | **50.0%** | 4.13 ± 0.09*** | 0.641 ± 0.012** | 2.66 ± 0.03*** | 4.97 ± 0.07 | 0.26 ± 0.01*** | 0.679 ± 0.015*** |
| | **6.25%** | 4.25 ± 0.03*** | 0.660 ± 0.015** | 2.20 ± 0.05*** | 5.26 ± 0.01*** | 0.32 ± 0.01*** | 0.797 ± 0.014*** |
| ***S*. Typhimurium ŽM352** | **Control** | 4.68 ± 0.21 | 0.496 ± 0.034 | 1.54 ± 0.10 | 6.13 ± 0.18 | 0.45 ± 0.03 | 0.590 + 0.034 |
| | **50.0%** | 4.26 ± 0.04* | 0.429 ± 0.017 | 1.51 ± 0.03 | 5.77 ± 0.06* | 0.46 ± 0.01 | 0.514 ± 0.033* |
| | **6.25%** | 4.33 ± 0.01* | 0.530 ± 0.10 | 1.52 ± 0.07 | 5.79 ± 0.07* | 0.46 ± 0.02 | 0.625 ± 0.015 |
| ***S*. Typhimurium ŽM375** | **Control** | 4.82 ± 0.03 | 0.657 ± 0.021 | 1.90 ± 0.17 | 5.96 ± 0.07 | 0.37 ± 0.03 | 0.863 ± 0.040 |
| | **50.0%** | 4.50 ± 0.01*** | 0.642 ± 0.001 | 2.58 ± 0.06** | 5.36 ± 0.02*** | 0.27 ± 0.01** | 0.702 ± 0.004** |
| | **6.25%** | 4.50 ± 0.01*** | 0.701 ± 0.019* | 2.05 ± 0.02 | 5.57 ± 0.02*** | 0.34 ± 0.01 | 0.846 ± 0.006* |
| ***S*. Hadar ŽM378** | **Control** | 4.54 ± 0.09 | 0.687 + 0.019 | 1.67 ± 0.10 | 5.85 ± 0.13 | 0.42 ± 0.03 | 0.987 ± 0.046 |
| | **50.0%** | 4.24 ± 0.01** | 0.665 ± 0.005 | 2.56 ± 0.12*** | 5.13 ± 0.02*** | 0.27 ±0.01*** | 0.718 ± 0.008*** |
| | **6.25%** | 4.34 ± 0.06* | 0.729 ± 0.002* | 2.07 ± 0.08** | 5.42 ± 0.01** | 0.34 ± 0.01** | 0.837 ± 0.004** |

| | | | | | | | |
|---|---|---|---|---|---|---|---|
| *, *p* <0.05; **, *p* <0.01; *** *p* <0.001; <control within strain (Dunett's t-tests) | | | | | | | |

**Table 3. Correlation coefficients (r) and significance (p) for the influence of the different dilutions of the Pleurotus ostreatus extract on Salmonella Infantis ŽM9.**

| | **End-point (OD₆₀₀)** | | **Crystal violet absorbance (a.u.)** | | **Number of biofilm cells (n)** | |
|---|---|---|---|---|---|---|
| | ***r*** | ***p*** | ***r*** | ***p*** | ***r*** | ***p*** |
| **End-point (OD₆₀₀)** | -- | -- | 0.873 | 0.005 | 0.912 | 0.002 |
| **Crystal violet absorbance (a.u.)** | 0.873 | 0.005 | -- | -- | 0.977 | <0.001 |
| **Number of biofilm cells (N)** | 0.912 | 0.002 | 0.977 | <0.001 | -- | -- |

### Screening of fungal extracts for antimicrobial and anti-biofilm activities against Salmonella Infantis

The details of the fungal extracts are given in Table 4. These extracts were analysed for their antimicrobial and anti-biofilm activities by evaluating: (i) the growth parameters (within the first 12 h); (ii) the end-point OD₆₀₀ (after 20 h); and (iii) the number of biofilm cells (after 20 h). All of these 42 fungal extracts tested showed some effects on bacterial growth and biofilm formation (Figure 3). In six fungal extracts, no growth parameters or end-point OD₆₀₀ could be obtained due to the strong coloration of the extracts at the 1:1 extract dilutions (50.0% relative concentration), although the numbers of biofilm cells were determined after 20 h. Complete growth inhibition was observed in fungal extracts from *Pseudohydnum gelatinosum* and *Infundibulicybe gibba.* For the other aqueous fungal extracts, however, the least effect was seen for the lag phase, as only two fungal extracts prolonged this phase (up to 1 h). There were larger effects on the other growth parameters, as 49% of the fungal extracts reduced the carrying capacity, and 83% reduced the growth rate (Figure 3A) and extended the doubling time. Lower end-point OD₆₀₀ values were seen for all of the extracts except from *Tricholoma sulphureum,* and there were lower numbers of biofilm cells for most of the fungal extracts, with significant decrease in 43% of the fungal extracts (Figure 3B, C). No differences between the activities of the wild and cultivated fungi were observed, nor were the bioactivities of the fungal extracts linked to any taxonomic families.

Complete growth inhibition of S. Infantis was achieved with *P. gelatinosum* and *I*. *gibba* extracts when diluted to 50.0% (1:1), where *I*. *gibba* also showed bactericidal effects. To see whether these two extracts only had antimicrobial effects or whether they also reduced biofilm formation, additional 2-fold dilutions were analyzed (to 1:16). As can be seen from the growth curves, both of these extracts at 1:4 dilution showed at least a doubled lag phase, with further bacterial growth also slowed down. Here, the growth-inhibition effects were more pronounced with these further dilutions for the *I*. *gibba* extract (Figure 4C). In agreement with the secondary increase in the growth curves and the number of biofilm cells determined (Figure 4A, B), *P. gelatinosum* inhibited growth at first two dilutions, which also reduced number of biofilm cells. But after no effect on growth was observed, there was no anti-biofilm activity. But *I*. *gibba* lowered the end-point OD₆₀₀ and reduced the number of biofilm cells in further dilutions where no effect on growth was observed (Figure 4C, D), thus showing potential anti-biofilm activity.

The other fungus of the genus *Infundibulicybe* that was tested here, *Infundibulicybe geotropa,* also significantly influenced all of the growth parameters, the end-point OD₆₀₀, and the number of biofilm cells (Figure 5A, B). Here too, we tested additional 2-fold dilutions, and while the influence on the growth parameters decreased with the higher dilutions used, the secondary increase in the growth curve was only seen at the 1:32 dilution (relative concentration 3.13%) and above, and the end-point OD₆₀₀ exceeded the value 1 at the 1:64 dilution (relative concentration 1.56%) (data not shown). Thus, while the influence on growth parameters and the antimicrobial activity was seen at the lower dilutions used (i.e., higher relative concentrations), anti-biofilm activity was seen across almost the full range of dilutions (to 1:64 dilution).

Since the antimicrobial activity of the aqueous extract from *I*. *geotropo* has been previously associated with L-amino acid oxidase (LAO) activity [26], we performed assays in the presence of catalase (Figure 5). The catalase had a noticeable effect on the change in the lag phase - it reversed the effect of the fungal extract making the growth curve comparable to the control. However, the inhibitory effect on the secondary increase of the growth curve was still present despite the addition of catalase, indicating the presence of other compounds that inhibit the biofilm formation of S. Infantis. Therefore, we analyzed the effect of the L-amino acid oxidase CgLAO that was purified from the *I*. *geotropo* extract and was previously shown to have antibacterial activity [26]. We compared the effect of CgLAO on the growth of S. Infantis to that of the *I*. *geotropo* extract (Figure 5). For both CgLAO and *I*. *geotropo* extract the effect on bacterial growth was concentration dependent. CgLAO showed antimicrobial effect against S. Infantis noticeable as concentration dependent lag phase prolongation (Figure 5C). In contrast to the effect observed with *I*. *geotropo* extract CgLAO had no effect on the secondary increase of the growth curve (Figure 5A, 5C) and nor on the number of biofilm cells (Figure 5D). This indicated the presence of other bioactive compounds in the *I*. *geotropo* extract that inhibit biofilm formation.

As indicated above, there was strong coloration of six of the extracts at the 1:1 extract dilutions, for which no growth parameters could be determined, although they showed more than 1.5 log units reductions in the number of biofilm cells. Here, three of these were selected for testing of further 1:1 dilutions: *Tylopilus felleus, Cortinarius violaceus,* and *Coprinus comatus.* In the extracts of *T. felleus* and *C. violaceus,* these absorbances (i.e., extract coloration) remained too high to record the growth curves even at the 1:16 dilution, while for *C. comatus* the growth curve could be plotted (data not shown). The inhibition by this further diluted *C. comatus* extract of the secondary increase in the growth curve and the end-point OD₆₀₀ were seen to decrease with increasing dilution of the extract, as also seen for the reduction of the number of biofilm cells. This greater inhibition of the number of biofilm cells at the lower dilutions (i.e., higher extract concentrations) was also seen for *T. felleus,* with a dose-related decrease in inhibition seen for the higher dilutions used (to 1:16; i.e., to the lowest tested relative concentration of 6.25%) that still showed significant reduction in the number of biofilm cells of 1.5 log units (data not shown). In *C. violaceus,* however, these effects were not concentration dependent, as the reduction of the number of biofilm cells remained constant even with the increasing dilutions (i.e., decreasing concentrations) of the extract (data not shown).

All of the other extracts at the 1:1 dilution also showed some significant effects on the parameters of the growth curves, with further significant differences seen for the end-point OD₆₀₀, and some significant decreases in the number of biofilm cells (Figure 3). These last effects (i.e., decreased numbers of biofilm cells) were small, but significant, in particular for the fungal extracts of *Amanita muscaria, Armilaria borealis,* and *Kuehneromyces mutabilis.* Indeed, as these extracts had a minimal influence on the growth curves and did not show pronounced growth inhibition, they showed only anti-biofilm activities, with >1 log unit reduction in the number of biofilm cells (Figure 3C).

**Table 4. Details of the fungal extracts used in this study, with the indications of their antimicrobial and anti-biofilm activities.**

| **Phylum** | **Family** | **Species** | **Fungal** source | **Dry** weight **(mg/mL)** | **Antibacterial activity** | **Anti-biofilm activity** |
|---|---|---|---|---|---|---|
| Ascomycota | Morchellaceae | *Leucangium carthusianum* | w | 16.15 | nd | - |
| | Tuberaceae | *Tuber aestivum* | w | 11.50 | - | + |
| | | *Tuber excavatum* | w | 6.00 | - | + |
| Basidomycota | Agaricaceae | *Agaricus bisporus* (brown) | c | 7.80 | + | + |
| | | *Agaricus bisporus* (white) | c | 12.65 | + | + |
| | | *Apioperdon pyriforme* | w | 15.17¹ | + | + |
| | | *Chlorophyllum rhacodes* | w | 23.55 | - | - |
| | | *Coprinus comatus* | w | 16.75¹ | + | - |
| | | *Macrolepiota procera* | w | 16.45¹ | + | + |
| | Amanitaceae | *Amanita citrina* | w | 73.00 | + | + |
| | | *Amanita excelsa* | w | 20.18¹ | + | + |
| | | *Amanita muscaria* | w | 7.70 | + | + |
| | | *Amanita rubescens* | w | 13.41¹ | + | + |
| | Auriculariales | *Pseudohydnum gelatinosum* | w | 2.10 | + | - |
| | Boletaceae | *Boletus reticulatus* | w | 1.70 | + | + |
| | | *Caloboletus calopus* | w | 8.65 | + | + |
| | | *Imleria badia* | w | 17.21¹ | nd | - |
| | | *Neoboletus erythropus* | w | 12.10 | nd | + |
| | | *Tylopilusfelleus* | w | 16.60 | + | - |
| | Cortinariaceae | *Cortinarius caperatus* | w | 15.30 | + | + |
| | | *Cortinarius violaceus* | w | 18.60 | nd | + |
| | Fomitopsidaceae | *Fomitopsis pinicola* | w | 15.90¹ | - | + |
| | Ganodermataceae | *Ganoderma lucidum* | c | 12.70 | - | + |
| | Lyophyllaceae | *Lyophyllum decastes* | w | 13.98¹ | - | + |
| | Omphalotaceae | *Lentinula edodes* | c | 9.90 | - | + |
| | Physalacriaceae | *Armilaria borealis* | w | 11.28¹ | + | + |
| | Pleurotaceae | *Pleurotus ostreatus* | c | 10.86¹ | - | + |
| | Rhizopogonaceae | *Rhizopogon verii* | w | 7.60 | nd | + |
| | Russulaceae | *Lactarius necator* | w | 13.92¹ | nd | - |
| | | *Lactarius vellereus* | w | 14.83¹ | + | + |
| | | *Russula alutacea* | w | 13.09¹ | + | + |
| | Sparassidaceae | *Sparassis crispa* | w | 14.68¹ | - | + |
| | Strophariaceae | *Kuehneromyces mutabilis* | w | 13.28¹ | + | + |
| | Suillaceae | *Suillus bovinus* | w | 3.20 | - | + |
| | | *Suillus granulatus* | w | 9.93¹ | + | + |
| | | *Suillus luteus* | w | 9.35 | + | + |
| | | *Suillus variegatus* | w | 15.24¹ | + | + |
| | Tapinellaceae | *Tapinella atromentosa* | w | 12.67¹ | - | + |
| | Tricholomataceae | *Clitocybe nebularis* | w | 27.11¹ | nd | - |
| | | *Infundibulicybe geotropo* | w | 28.93¹ | + | + |
| | | *Infundibulicybe gibba* | w | 20.02¹ | + | + |
| | | *Tricholoma pardinum* | w | 11.25 | + | + |
| | | *Tricholoma sulphureum* | w | 14.12¹ | + | - |

| | | | | | | |
|---|---|---|---|---|---|---|
| w, wild; c, cultivated; nd, not determined; +, positive; -, negative 1 Dry weights refer to Klančnik et al. [23]. | | | | | | |

### Discussion

Here, we have described the successful use of growth curve analysis to simultaneously evaluate antibacterial and anti-biofilm activities as a high-throughput analysis for the testing of new potentially active substances (Figure 6).

Out of four *Salmonella* serovars, S. Infantis was selected as it showed the greatest changes in the growth curve, and particularly for the secondary increase in the growth curve. This was confirmed to be a consequence of the formation of biofilm biomass, which is probably due to EPS production. By defining the bacterial growth according to the growth curve, it is possible to evaluate whether tested compounds show antibacterial and/or anti-biofilm activities - antibacterial in terms of an effect on growth, and anti-biofilm in terms of an effect on production of biofilm biomass. Although antibacterial compounds can reduce biofilm production, it is important to distinguish between these two concepts.

With the use of spectrophotometric detection of the growth of S. Infantis, we can distinguish between growth-inhibiting and biofilm-inhibiting activities (Figure 6). The effects on the first phase of the curve - *i.e.,* the lag phase, growth rate, doubling time and carrying capacity - represents the influence of an active compound on the growth of the bacteria. The effects on the second phase of the curve - *i.e.,* the secondary increase in the growth curve - represents the influence of an active compound on the biofilm formation of the bacteria.

With the proposed approach, we present a simple and rapid way to distinguish between antibacterial and anti-biofilm activities. This allows the testing of larger numbers of potentially active compounds at once, with the results obtained within 20 h. This is suitable for solutions of active compounds where the initial absorbance at 600 nm is no more than 0.500. Furthermore, this approach allows one to determine which bioactive compound has anti-biofilm activity, in addition to antimicrobial activity.

### List of references cited in the description

[1] MacKenzie, K. D., Palmer, M. B., Köster, W. L. & White, A. P. Examining the link between biofilm formation and the ability of pathogenic Salmonella strains to colonize multiple host species. Front. Vet. Sci. 4, 138 (2017).
[2] Flemming, H.-C. & Wingender, J. The biofilm matrix. Not. Rev. Microbiol. 8, 623-633 (2010).
[3] González, J. F., Alberts, H., Lee, J., Doolittle, L. & Gunn, J. S. Biofilm formation protects Salmonella from antibiotic ciprofloxacin in vitro and in vivo in the mouse model of chronic carriage. Sci. Rep. 8, 222 (2018).
[4] Tassinari, E. et al. Microevolution of antimicrobial resistance and biofilm formation of Salmonella Typhimurium during persistence on pig farms. Sci. Rep. 9, 8832 (2019).
[5] Steenackers, H., Hermans, K., Vanderleyden, J. & De Keersmaecker, S. C. J. Salmonella biofilms: an overview on occurrence, structure, regulation and eradication. Food Res. Int. 45, 502-531 (2012).
[6] EFSA & ECDC. The European Union one health 2018 zoonoses report. EFSA J. 17, 5926 (2019).
[7] EFSA & ECDC. The European Union summary report on antimicrobial resistance in zoonotic and indicator bacteria from humans, animals and food in 2017/2018. EFSA J. 18, 6007 (2020).
[8] Pate, M., Mičunovi 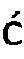 , J., Golob, M., Vestby, L. K. & Ocepek, M. *Salmonella* Infantis in broiler flocks in Slovenia: the prevalence of multidrug resistant strain with high genetic homogeneity and low biofilm-forming ability. *BioMed Res. Int.* **2019,** 4981463 (2019).
[9] Vestby, L. K., Møretrø, T., Langsrud, S., Heir, E. & Nesse, L. L. 2009. Biofilm forming ability of Salmonella are correlated with persistance in fish meal- and feed factories. BMC Vet. Res. 5, 20 (2009).
[10] Adetoye, A., Pinloche, E., Adeniyi, B. A. & Ayeni, F. A. Characterization and anti-salmonella activities of lactic acid bacteria isolated from cattle faeces. BMC Microbiol. 18, 96 (2018).
[11] Cooper, I. R. A review of current methods using bacteriophages in live animals, food and animal products intended for human consumption. J. Microbiol. Methods 130, 38-47 (2016).
[12] Moshiri, J. et al. Identification of a small molecule anti-biofilm agent against Salmonella enterica. Front. Microbiol. 9, 2804 (2018).
[13] Reid, T. et al. Characterization of anti-Salmonella typhi compounds from medicinal mushroom extracts from Zimbabwe. Int. J. Med. Mushrooms 21, 713-724 (2019).
[14] Santos, S. A. O., Martins, C., Pereira, C., Silvestre, A. J. D. & Rocha, S. M. Current challenges and perspectives for the use of aqueous plant extracts in the management of bacterial infections: the case-study of Salmonella enterica serovars. Int. J. Mol. Sci. 20, 940 (2019).
[15] Dieltjens, L. et al. Inhibiting bacterial cooperation is an evolutionarily robust anti-biofilm strategy. Not. Commun. 11, 107 (2020).
[16] Klančnik, A., Piskernik, S., Jeršek, B. & Smole Možina, S. 2010. Evaluation of diffusion and dilution methods to determine the antibacterial activity of plant extracts. *J*. *Microbiol. Methods* **81,** 121-126 (2010).
[17] Balouiri, M., Sadiki, M. & Ibnsouda, S. K. Methods for in vitro evaluating antimicrobial activity: a review. J. Pharm. Anal. 6, 71-79 (2016)
[18] Klančnik, A. et al. Anti-Campylobacter activity of resveratrol and an extract from waste Pinot noir grape skins and seeds, and resistance of *Comp. jejuni* planktonic and biofilm cells, mediated via the CmeABC efflux pump. *J*. *Appl. Microbiol.* **122,** 65-77 (2016).
[19] Àlvarez, G., González, M., Isabal, S., Blanc, V. & León, R. Method to quantify live and dead cells in multi-species oral biofilm by real-time PCR with propidium monoazide. AMB Express 3, 1 (2013).
[20] Klančnik, A. et al. 2018. Antiadhesion activity of juniper (*Juniperus communis* L.) preparations against *Campylobacter jejuni* evaluated with PCR-based methods. *Phytother. Res.* **32,** 542-550 (2018).
[21] Cerca, F. et al. SYBR green as a fluorescent probe to evaluate the biofilm physiological state of Staphylococcus epidermidis, using flow cytometry. Con. J. Microbiol. 57, 850-856 (2011).
[22] Kerstens, M. et al. A flow cytometry approach to quantify biofilms. Folio Microbiol. 60, 335-342 (2015).
[23] Klančnik, A. et al. Aqueous extracts of wild mushrooms show antimicrobial and antiadhesion activities against bacteria and fungi. *Phytother. Res.* **31,** 1971-1976 (2017).
[24] Sprouffske, K. & Wagner, A. Growthcurver: an R package for obtaining interpretable metrics from microbial growth curves. BMC Bioinform. 17, 172 (2016).
[25] Baranyi, J. &Roberts, T. A. A dynamic approach to predicting bacterial growth in food. Int. J. Food Microbiol. 23, 277-294 (1994).
[26] Sabotič, J. et al. L - amino acid oxidases from mushrooms show antibacterial activity against the phytopathogen *Ralstonia solanacearum. Front. Microbiol.* **11,** 977 (2020).

## Claims

1. A method for identifying a composition or compound having antimicrobial and/or anti-biofilm activities against a microorganism of interest, comprising the steps of a) providing a composition or compound, b) bringing the composition or compound in contact with a microorganism of interest in a growth medium; c) incubating said microorganism in the presence of said composition or compound; d) determining the growth of the microorganism by measuring the optical density in intervals during the incubation under c); e) preparing a growth curve from the measurement obtained under d); and f) analyzing the growth curve to determine if the composition or compound has antimicrobial and/or anti-biofilm activities against the microorganism;
wherein step f) comprises comparing the growth curve prepared in step e) with a control growth curve which is obtained in accordance with steps c) to e) with all conditions being identical except that the microorganism is incubated in the absence of the composition or compound;
wherein the control growth curve exhibits a first phase including an increase in optical density within a first period of time, a second phase including an increase in optical density within a second period of time, and a stationary phase between the first phase and the second phase.

2. The method according to claim 1, wherein the composition or compound is brought in contact with the microorganism in step b) by mixing an inoculum of the microorganism in growth medium with the composition or compound.

3. The method according to claim 1 or 2, wherein the composition or compound is considered having antimicrobial activity against the microorganism if the growth curve, when compared to the control growth curve, shows a decrease in the optical density in the first phase within the first period of time.

4. The method according to any one of claims 1 to 3, wherein the composition or compound is considered having antimicrobial activity against the microorganism if the growth curve, when compared to the control growth curve, shows a decrease of at least 10% in the optical density in the first phase within the first period of time.

5. The method according to any one of claims 1 to 4, wherein step f) comprises evaluating at least one growth parameter for the first phase.

6. The method according to claim 5, wherein the at least one growth parameter is selected from the group consisting of lag phase length, carrying capacity, growth rate, doubling time, time in which the population density reaches half of the carrying capacity, and end-point optical density (OD).

7. The method according to claim 5 or 6, wherein the composition or compound is considered having antimicrobial activity against the microorganism if at least one parameter in the growth curve selected from the group consisting of carrying capacity, growth rate, and end-point optical density (OD) is lower than the corresponding parameter in the control growth curve.

8. The method according to any one of claims 5 to 7, wherein the composition or compound is considered having antimicrobial activity against the microorganism if at least one parameter in the growth curve selected from the group consisting of the lag phase length, doubling time, and time in which the population density reaches half of the carrying capacity is longer than the corresponding parameter in the control growth curve.

9. The method according to any one of claims 1 to 8, wherein the composition or compound is considered having anti-biofilm activity against the microorganism if the growth curve, when compared to the control growth curve, shows a decrease in the optical density in the second phase within the second period of time.

10. The method according to any one of claims 1 to 9, wherein step f) further comprises evaluating the end-point optical density (OD) for the second phase.

11. The method according to claim 10, wherein the composition or compound is considered having anti-biofilm activity against the microorganism if the end-point OD in the growth curve is lower than the end-point OD in the control growth curve.

12. The method according to any one of claims 1 to 11, wherein the microorganism of interest is a bacterium or a yeast.

13. The method according to any one of claims 1 to 12, wherein the composition or compound is derived from or has been isolated from a natural source.

14. The method according to any one of claims 1 to 12, wherein the composition is an extract or a fraction thereof derived from a fungus, plant, algae, archaea, bacterium or animal cell or tissue.

15. The method according to any one of claims 1 to 12, wherein the composition comprises one or more chemically synthesized compounds.

## Patentansprüche

1. Verfahren zum Identifizieren einer Zusammensetzung oder Verbindung mit antimikrobieller und/oder Anti-Biofilm-Aktivität gegen einen bestimmten Mikroorganismus, umfassend die Schritte a) des Bereitstellens einer Zusammensetzung oder Verbindung; b) des Inkontaktbringens der Zusammensetzung oder Verbindung mit einem bestimmten Mikroorganismus in einem Wachstumsmedium; c) des Inkubierens des Mikroorganismus in der Gegenwart der Zusammensetzung oder Verbindung; d) des Bestimmens des Wachstums des Mikroorganismus durch Messen der optischen Dichte in Intervallen während der Inkubation unter c); e) des Erstellens einer Wachstumskurve aus der unter d) erlangten Messung; und f) des Analysierens der Wachstumskurve, um zu bestimmen, ob die Zusammensetzung oder Verbindung eine antimikrobielle und/oder Anti-Biofilm-Aktivität gegen den Mikroorganismus aufweist;
wobei Schritt f) Vergleichen der in Schritt e) erstellten Wachstumskurve mit einer Kontrollwachstumskurve umfasst, die gemäß den Schritten c) bis e) mit komplett identischen Bedingungen, außer dass der Mikroorganismus in Abwesenheit der Zusammensetzung oder Verbindung inkubiert wird, erlangt wird;
wobei die Kontrollwachstumskurve eine erste Phase, die eine Zunahme der optischen Dichte innerhalb eines ersten Zeitraums beinhaltet, eine zweite Phase, die eine Zunahme der optischen Dichte innerhalb eines zweiten Zeitraums beinhaltet, und eine stationäre Phase zwischen der ersten Phase und der zweiten Phase aufweist.

2. Verfahren nach Anspruch 1, wobei die Zusammensetzung oder Verbindung in Schritt b) mit dem Mikroorganismus in Kontakt gebracht wird, indem ein Inokulum des Mikroorganismus im Wachstumsmedium mit der Zusammensetzung oder der Verbindung vermischt wird.

3. Verfahren nach Anspruch 1 oder 2, wobei die Zusammensetzung oder Verbindung als antimikrobielle Aktivität gegen den Mikroorganismus aufweisend betrachtet wird, wenn die Wachstumskurve im Vergleich mit der Kontrollwachstumskurve eine Abnahme der optischen Dichte in der ersten Phase innerhalb des ersten Zeitraums zeigt.

4. Verfahren nach einem der Ansprüche 1 bis 3, wobei die Zusammensetzung oder Verbindung als antimikrobielle Aktivität gegen den Mikroorganismus aufweisend betrachtet wird, wenn die Wachstumskurve im Vergleich mit der Kontrollwachstumskurve eine Abnahme von mindestens 10 % in der optischen Dichte in der ersten Phase innerhalb des ersten Zeitraums zeigt.

5. Verfahren nach einem der Ansprüche 1 bis 4, wobei Schritt f) Bewerten mindestens eines Wachstumsparameters für die erste Phase umfasst.

6. Verfahren nach Anspruch 5, wobei der mindestens eine Wachstumsparameter ausgewählt ist aus der Gruppe bestehend aus Anlaufphasenlänge, Aufnahmefähigkeit, Wachstumsrate, Verdopplungszeit, der Zeit, in der die Populationsdichte die Hälfte der Aufnahmefähigkeit erreicht, und einem Endpunkt der optischen Dichte (OD).

7. Verfahren nach Anspruch 5 oder 6, wobei die Zusammensetzung oder Verbindung als antimikrobielle Aktivität gegen den Mikroorganismus aufweisend betrachtet wird, wenn mindestens ein Parameter in der Wachstumskurve, der ausgewählt ist aus der Gruppe bestehend aus Aufnahmefähigkeit, Wachstumsrate und Endpunkt der optischen Dichte (OD), geringer als der entsprechende Parameter in der Kontrollwachstumskurve ist.

8. Verfahren nach einem der Ansprüche 5 bis 7, wobei die Zusammensetzung oder Verbindung als antimikrobielle Aktivität gegen den Mikroorganismus aufweisend betrachtet wird, wenn mindestens ein Parameter in der Wachstumskurve, der ausgewählt ist aus der Gruppe bestehend aus der Anlaufphasenlänge, Verdopplungszeit und der Zeit, in der die Populationsdichte die Hälfte der Aufnahmefähigkeit erreicht, länger als der entsprechende Parameter in der Kontrollwachstumskurve ist.

9. Verfahren nach einem der Ansprüche 1 bis 8, wobei die Zusammensetzung oder Verbindung als Anti-Biofilm-Aktivität gegen den Mikroorganismus aufweisend betrachtet wird, wenn die Wachstumskurve im Vergleich mit der Kontrollwachstumskurve eine Abnahme in der optischen Dichte in der zweiten Phase innerhalb des zweiten Zeitraums zeigt.

10. Verfahren nach einem der Ansprüche 1 bis 9, wobei Schritt f) ferner Bewerten des Endpunkts der optischen Dichte (OD) für die zweite Phase umfasst.

11. Verfahren nach Anspruch 10, wobei die Zusammensetzung oder Verbindung als Anti-Biofilm-Aktivität gegen den Mikroorganismus aufweisend betrachtet wird, wenn der OD-Endpunkt in der Wachstumskurve geringer als der OD-Endpunkt in der Kontrollwachstumskurve ist.

12. Verfahren nach einem der Ansprüche 1 bis 11, wobei der bestimmte Mikroorganismus ein Bakterium oder eine Hefe ist.

13. Verfahren nach einem der Ansprüche 1 bis 12, wobei die Zusammensetzung oder Verbindung von einer natürlichen Quelle abgeleitet ist oder von dieser isoliert wurde.

14. Verfahren nach einem der Ansprüche 1 bis 12, wobei die Zusammensetzung ein Extrakt oder eine Fraktion davon ist, das/die von einem Pilz, einer Pflanze, einer Alge, einem Archaeon, einem Bakterium oder einer Tierzelle oder Tiergewebe abgeleitet ist.

15. Verfahren nach einem der Ansprüche 1 bis 12, wobei die Zusammensetzung eine oder mehrere chemisch synthetisierte Verbindungen umfasst.

## Revendications

1. Procédé d'identification d'une composition ou d'un composé ayant des activités antimicrobiennes et/ou anti-biofilm contre un micro-organisme d'intérêt, comprenant les étapes a) de fourniture d'une composition ou d'un composé, b) de mise en contact de la composition ou du composé avec un micro-organisme d'intérêt dans un milieu de croissance ; c) d'incubation dudit micro-organisme en présence de ladite composition ou dudit composé ; d) de détermination de la croissance du micro-organisme en mesurant la densité optique à des intervalles pendant l'incubation en c) ; e) de préparation d'une courbe de croissance à partir de la mesure obtenue en d) ; et f) d'analyse de la courbe de croissance pour déterminer si la composition ou le composé a des activités antimicrobiennes et/ou anti-biofilm contre le micro-organisme ;
dans lequel l'étape f) comprend la comparaison de la courbe de croissance préparée à l'étape e) avec une courbe de croissance témoin qui est obtenue conformément aux étapes c) à e), toutes les conditions étant identiques sauf que le micro-organisme est incubé en l'absence de la composition ou du composé ;
dans lequel la courbe de croissance témoin présente une première phase comportant une augmentation de la densité optique au cours d'une première période de temps, une seconde phase comportant une augmentation de la densité optique au cours d'une seconde période de temps, et une phase stationnaire entre la première phase et la seconde phase.

2. Procédé selon la revendication 1, dans lequel la composition ou le composé est mis en contact avec le micro-organisme à l'étape b) en mélangeant un inoculum du micro-organisme dans un milieu de croissance avec la composition ou le composé.

3. Procédé selon la revendication 1 ou 2, dans lequel la composition ou le composé est considéré comme ayant une activité antimicrobienne contre le micro-organisme si la courbe de croissance, lorsqu'on la compare à la courbe de croissance témoin, montre une diminution de la densité optique dans la première phase au cours de la première période de temps.

4. Procédé selon l'une quelconque des revendications 1 à 3, dans lequel la composition ou le composé est considéré comme ayant une activité antimicrobienne contre le micro-organisme si la courbe de croissance, lorsqu'on la compare à la courbe de croissance témoin, montre une diminution d'au moins 10 % de la densité optique dans la première phase au cours de la première période de temps.

5. Procédé selon l'une quelconque des revendications 1 à 4, dans lequel l'étape f) comprend l'évaluation d'au moins un paramètre de croissance pour la première phase.

6. Procédé selon la revendication 5, dans lequel l'au moins un paramètre de croissance est sélectionné dans le groupe constitué de la longueur de phase de latence, de la capacité de charge, du taux de croissance, du temps de doublement, du temps pendant lequel la densité de population atteint la moitié de la capacité de charge, et de la densité optique (OD) de point d'extrémité.

7. Procédé selon la revendication 5 ou 6, dans lequel la composition ou le composé est considéré comme ayant une activité antimicrobienne contre le micro-organisme si au moins un paramètre dans la courbe de croissance sélectionné dans le groupe constitué de la capacité de charge, du taux de croissance et de la densité optique (OD) de point d'extrémité est inférieur au paramètre correspondant dans la courbe de croissance témoin.

8. Procédé selon l'une quelconque des revendications 5 à 7, dans lequel la composition ou le composé est considéré comme ayant une activité antimicrobienne contre le micro-organisme si au moins un paramètre dans la courbe de croissance sélectionné dans le groupe constitué de la longueur de phase de latence, du temps de doublement et du temps pendant lequel la densité de population atteint la moitié de la capacité de charge est supérieur au paramètre correspondant dans la courbe de croissance témoin.

9. Procédé selon l'une quelconque des revendications 1 à 8, dans lequel la composition ou le composé est considéré comme ayant une activité anti-biofilm contre le micro-organisme si la courbe de croissance, lorsqu'on la compare à la courbe de croissance témoin, montre une diminution de la densité optique au cours de la seconde phase au cours de la seconde période.

10. Procédé selon l'une quelconque des revendications 1 à 9, dans lequel l'étape f) comprend en outre l'évaluation de la densité optique (OD) de point d'extrémité pour la seconde phase.

11. Procédé selon la revendication 10, dans lequel la composition ou le composé est considéré comme ayant une activité anti-biofilm contre le micro-organisme si la OD de point d'extrémité dans la courbe de croissance est inférieure à la OD de point d'extrémité dans la courbe de croissance témoin.

12. Procédé selon l'une quelconque des revendications 1 à 11, dans lequel le micro-organisme d'intérêt est une bactérie ou une levure.

13. Procédé selon l'une quelconque des revendications 1 à 12, dans lequel la composition ou le composé est dérivé ou a été isolé d'une source naturelle.

14. Procédé selon l'une quelconque des revendications 1 à 12, dans lequel la composition est un extrait ou une fraction de celui-ci dérivé d'un champignon, d'une plante, d'une algue, d'une archée, d'une bactérie ou d'une cellule ou d'un tissu animal.

15. Procédé selon l'une quelconque des revendications 1 à 12, dans lequel la composition comprend un ou plusieurs composés chimiquement synthétisés.
